(19) Europäisches Patentamt
European Patent Office
Office européen des brevets

(11) **EP 2 085 486 A1**

(12) **EUROPEAN PATENT APPLICATION**

(43) Date of publication:
**05.08.2009 Bulletin 2009/32**

(51) Int Cl.:
*C12Q 1/68* $^{(2006.01)}$

(21) Application number: **08290090.3**

(22) Date of filing: **01.02.2008**

| | |
|---|---|
| (84) Designated Contracting States: **AT BE BG CH CY CZ DE DK EE ES FI FR GB GR HR HU IE IS IT LI LT LU LV MC MT NL NO PL PT RO SE SI SK TR** Designated Extension States: **AL BA MK RS** | (72) Inventors: • **Payen de la Garanderie, Didier 75018 Paris (FR)** • **Lukaszewicz, Anne-Claire 60500 Chantilly (FR)** |
| (71) Applicant: **Assistance Publique - Hôpitaux de Paris 75004 Paris (FR)** | (74) Representative: **Marcadé, Véronique et al Cabinet Ores 36, rue de St Pétersbourg 75008 Paris (FR)** |

(54) **Methods and kits for the rapid determination of patients at high risk of death during septic shock**

(57) The present invention relates to the field of treatment of serious medical syndromes such as severe sepsis and septic shock. In particular, the present invention provides methods and kits to obtain an early evaluation of mortality risk and help therapeutic decisions for patients in severe sepsis with two organ failures, for example for patients in septic shock with one additional organ failure. The methods of the invention are based on the analysis of an expression profile of one or several genes selected in the group consisting of HLA-DRB4, FOSB, GPR109B, RBP7, TLR7, IL15, HLA-C, AMFR, LOC96610, IRF5, MGC29506, EDG3, IGLV1-44, IFIT2, IFI44, EPSTI1, TNFRSF17, AREG, THBS1, CTLS1, TFPI, PHACTR2, PFKFB2 and CHIT1.

EP 2 085 486 A1

SEQUENCE LISTING

<110>  ASSISTANCE PUBLIQUE - HOPITAUX DE PARIS
       PAYEN DE LA GARANDERIE , Didier
       LUKASZEWICZ, Anne-Claire

<120>  METHODS AND KITS FOR THE RAPID DETERMINATION OF PATIENTS AT HIGH
       RISK OF DEATH DURING SEPTIC SHOCK

<130>  VMAahF1020/27EP

<160>  3

<170>  PatentIn version 3.3

<210>  1
<211>  25
<212>  DNA
<213>  Artificial

<220>
<223>  Forward primer

<400>  1
tttgtgcttc cctttaccta aactg                                    25

<210>  2
<211>  23
<212>  DNA
<213>  Artificial

<220>
<223>  Reverse primer

<400>  2
aataatgcaa tgtgtggcac aag                                      23

<210>  3
<211>  25
<212>  DNA
<213>  Artificial

<220>
<223>  Probe

<400>  3
cctgcctccc gtgcatctgt actcc                                    25

**Description**

## FIELD OF THE INVENTION

The present invention relates to the field of treatment of serious medical syndromes such as severe sepsis or septic shock. In particular, the present invention provides methods and kits to obtain an early evaluation of mortality risk and help therapeutic decisions for patients in severe sepsis with two organ failures, for example for patients in septic shock with one additional organ failure.

## BACKGROUND AND PRIOR ART

Septic shock is the most severe clinical presentation of sepsis, with a poor prognosis despite intensive therapeutic support and anti-infectious strategy to eradicate the infection foci. The sepsis syndrome is defined as symptoms related to the host response to abnormal presence of micro-organisms or their antigenic fractions. The local infection might spread out for different reasons to the whole body, with a particular activation of blood immune cells controlling the innate immunity during the early phase and activation of the adaptive immunity in a second time. Such an intense immune activation in blood may in turn target organs that were not initially concerned by the initial infection, leading to immune toxicity and dysfunction of these organs. The high mortality rate of septic shock (around 50%) results from a combination of organ failures, comorbidities and virulence of micro-organisms. Death may occur at different times of evolution, most often during the first week despite intensive resuscitation.

Treatment of septic shock primarily consists of antimicrobial chemotherapy, removal of the source of infection when it is possible, and support of organ failure. Other proposed pathophysiological therapies are still under evaluation. In septic shock, corticosteroids, especially if combined with a mineralocorticoid, could reduce mortality among patients who have relative adrenal insufficiency (Annane and Bellissant, 2000). In patients with multi-organ failure (for example, septic shock plus one additional organ failure), only Activated Protein C (*APC*, also called drotrecogin alpha) has proven its efficacy in a randomized clinical trial, despite an increased risk of serious bleeding (Bernard et al., 2001). However, the cost and potential risk of this innovative drug have limited its wide use.

Until now, no biological markers have been demonstrated to predict reliably the patient outcome at the initial day of septic shock. To date, it is hence impossible to distinguish at a very early stage patients who really need expensive high tech drugs from patients for whom over treatment must be avoided. Since no clinical or routine biological parameters may help to discriminate patients in term of outcome for a given severity, having a rapid test for such a characterization would be of great help for the therapeutic strategy making.

In the last decade, the explosive development and use of genetic techniques applied in sepsis have brought information on sepsis susceptibility in relation to different genotypes. Such an approach is important but does not allow applying a specific strategy, since this constitutive genotype cannot be modified. The recent development of functional genomic methods becomes a tool to analyze the functionality of the genes, especially when pangenomic microchips are used. Such a technique has been used to define prognostic footprint in cancer (van 't Veer et al., 2002). Recently, this approach was used to analyze changes in blood leukocyte gene expression patterns in healthy human subjects receiving an inflammatory stimulus (bacterial endotoxin). Human blood leukocyte response to acute systemic inflammation includes the transient dysregulation of leukocyte bioenergetics (Belikova et al., 2007) and modulation of translational machinery (Calvano et al., 2005).

## SUMMARY OF THE INVENTION

In this context, the inventors have made the hypothesis that a limited number of positive or negative gene expression modifications in blood white cells may enable to identify patients at high risk of death, in comparison with patients with good prognosis. By using the micro-array technology performed with a pangenomic microchip on white blood cells of 48 patients having a septic shock, the inventors have identified a profile of early gene expression which is indicative of a good or a poor prognosis. As shown below, these results have been validated by real time PCR applied to some of the found genes of interest. For one of these genes, encoding the HLA-DRB4 molecule, the gene expression differed so strongly at day 0 between survivals and dead patients that a genotyping of this gene was performed. This showed the presence or the absence of this gene according to its expression.

The present invention hence provides an early biological footprint or profile indicative of outcome of patients in severe sepsis with at least two organ failures, and in particular, for patients in septic shock with at least one additional organ failure. This footprint can be obtained by microarray or by any other technique, such as real time PCR, which is the most efficient one to date, in terms of sensitivity and rapidity (a few hours). The status of HLA-DRB4 can also be determined by genotyping. A genomic and/or genotyping test according to the present invention can be used for outcome prediction in a few hours after the onset of septic shock, but also to determine which patients will be candidate to receive innovative drugs. This later pharmacogenomic aspect might have an important impact on the cost of septic shock treatment, since these innovative drugs will be more adequately used. The test can also be used to check a patient's response to a given treatment.

## BRIEF DESCRIPTION OF THE DRAWINGS

**Figure 1** shows the principal component analysis (PCA) at day 0, which enables to display patients into a graphic according to their similarities in expression profiles. Black spots represent non surviving patients and grey spots represent surviving patients (n=48 patients).

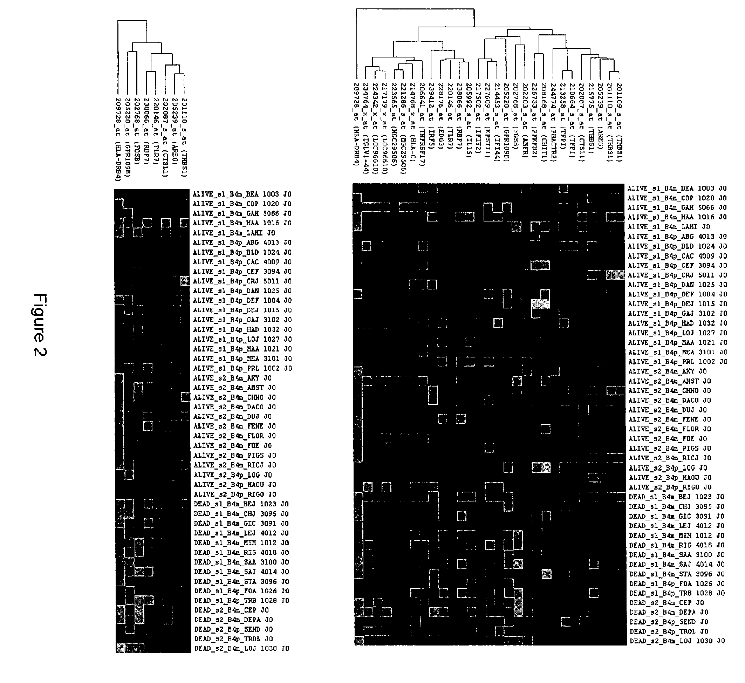

**Figure 2** shows the dendrogram of gene expression profile at day 0 after classification of dead versus alive patients (n=48) at day 0. The upper part shows the dendrogram for 29 sets of probes, the lower part the dendrogram for 8 sets of probes. Probes sets intensities were selected according to criteria FC>2 and p-value <0.1.

**Figure 3** shows the diagram of Kaplan-Meier from day 0 to day 28 according to the B4 expression status. It shows a dramatic difference in mortality between HLA-DRB4+ and HLA-DRB4- gene expression at day 0.

**Figure 4** shows the ROC (Receiving Operator Characteristics) curves generated by cross validation and represents the specificity (Sp) and sensibility (Ss) according to the chosen threshold for cutting in the model built with 29 (A) or 8 (B) sets of probes.

## DETAILED DESCRIPTION OF THE PREFERRED EMBODIMENTS

In the present text, the following definitions are used:

- a "severe sepsis" is defined as a sepsis syndrome with at least one organ dysfunction;
- a "septic shock" is defined as a severe sepsis syndrome with hypotension requiring vasopressors (which constitutes a first organ failure);
- day 0 designates the 24-hours period of severe sepsis after the onset of two organ failures; in the particular case of septic shock, day 0 hence designates the 24-hours period of septic shock after the onset of a second organ failure;
- "HLA-DRB4" designates, depending on the context, either the protein or the gene encoding it. As a genetic prognosis marker, HLA-DRB4 can be detected either at the genomic level (DNA), or at the mRNA level, or at the protein level. In what follows, when this or other genes of interest are cited, the same notation is used for the gene and the protein which it encodes, it being understood that the level of expression of a gene can be measured either at the transcription level (mRNA), or at the translation level (protein).
- a "representative cohort" is a cohort of patients who have undergone a severe sepsis with at least two organ failures, wherein the outcome of said severe sepsis is known. A representative cohort must comprise at least 40 patients, with at least 10 in each group (surviving *vs*/ dead). The patients must have been cared according to the recommendations of the Surviving Sepsis Campaign (Dellinger et al., 2008). Of course, the skilled artisan can chose to use a more numerous cohort for implementing the present invention, and the members of the representative cohort must have been chosen without any selection bias.
- in what follows, and except in particular cases for which a different definition is specified (*e.g.*, when data from a patient are compared to earlier data obtained in the same patient), "over-expression" and "under-expression" of a gene in a subject is determined by comparing the level of expression of said gene to the mean expression of the same gene in a representative cohort (including all the subjects of the cohort), it being understood that the level of expression of said gene in said subject is measured by using the same technique as was used for measuring the level of expression of the same gene in the subjects of the cohort. A gene will then be considered as over-expressed if its expression is superior (fold change > 1.2) to the mean expression in the representative cohort, and under-expressed if it is inferior (fold change < -1.2) to said mean expression.

According to a first embodiment, the invention pertains to the use of one gene or a set of two, three, four or more genes selected amongst HLA-DRB4 (human leucocyte antigen-DRB4), FOSB (FBJ murine osteosarcoma viral oncogene homolog B), GPR109B (G-protein coupled receptor, HM74), RBP7 (retinol binding protein 7), TLR7 (Toll like receptor 7), AREG (amphiregulin), THBS1 (thrombospondin 1), CTSL1 (cathepsin L1), IL15 (interleukin 15), HLA-C (major histocompatibility complex, class I, C), AMFR (autocrine motility factor receptor), LOC96610 (hypothetical protein similar to KIAA0187 gene product), IRF5 (interferon regulatory factor 5), MGC29506 (hypothetical protein MGC29506), EDG3 (endothelial differentiation, sphingolipid G-protein-coupled receptor, 3), IGLV 1-44 (immunoglobulin lambda variable 1 - 44), IFIT2 (interferon-induced protein with tetratricopeptide repeats 2), IFI44 (interferon-induced protein 44), EPSTI1 (epithelial stromal interaction 1), TNFRSF17 (tumor necrosis factor receptor superfamily, member 17), TFPI (tissue factor pathway inhibitor), PHACTR2 (phosphatase and actin regulator 2), PFKFB2 (6-phosphofructo-2-kinase/fructose-2,6-biphosphatase 2) and CHIT1 (chitinase 1), as a prognosis marker for a patient in severe sepsis with at least two organ failures. The use of the same gene or set of genes, as a marker for helping a physician in determining a therapeutic strategy and/or in determining the impact of a drug for a patient in severe sepsis with at least two organ failures is also part of the present invention. In a preferred embodiment, at least one, two or three of said gene(s) is/are selected amongst HLA-DRB4, AREG, FOSB, GPR109B, RBP7, TLR7, THBS1 and CTLS1, and even more preferably in the group consisting of HLA-DRB4, AREG and FOSB.

As described in more details below, up-regulation of one or several genes selected amongst HLA-DRB4, FOSB, GPR109B, RBP7, TLR7, IL15, HLA-C, AMFR, LOC96610, IRF5, MGC29506, EDG3, IGLV1-44, IFIT2, IFI44, EPSTI1 and TNFRSF17, and/or down-regulation of one or several genes selected amongst AREG, THBS1, CTLS1, TFPI, PHACTR2, PFKFB2 and CHIT1, as measured in a patient in severe sepsis with at least two organ failures, at day 0, are indicative of a good prognosis.

Since severe sepsis is a very heterogeneous syndrome, it can be more relevant to perform a coordinate analysis of several genes, rather than looking at the expression level of only one gene.

Accordingly, the present invention also concerns a method for *in vitro* establishing a prognosis for a subject in severe sepsis with at least two organ failures, comprising the following steps:

> (i) from a sample from said subject, obtaining an expression profile of a set of genes, wherein said set of genes comprises at least two genes selected amongst HLA-DRB4, FOSB, GPR109B, RBP7, TLR7, IL15, HLA-C, AMFR, LOC96610, IRF5, MGC29506, EDG3, IGLV1-44, IFIT2, IFI44, EPSTI1, TNFRSF17, AREG, THBS1, CTLS1, TFPI, PHACTR2, PFKFB2 and CHIT1; and
> (ii) comparing said obtained expression profile to one or two reference expression profile(s) to establish a prognosis for said subject.

For performing the above method, the reference expression profile(s) is (are) obtained from a representative cohort. Of course, the same technology will be used to establish the reference profile(s) and to obtain the expression profile of a tested subject.

When two reference expression profiles are used, one of them is obtained from the subjects of said cohort who have died from their severe sepsis, while other reference profile is obtained from the subjects of said cohort who have survived. In this case, the expression profile of the tested subject is compared to each of these reference profiles, in order to establish a prognosis. Many statistical techniques have been described for performing such comparisons, and the skilled artisan is free to chose any of them.

When only one reference profile is used, it is obtained from all the subjects of a representative cohort. For example, the reference profile can be obtained by determining, for each gene of the considered set of genes, the mean expression level of said gene in the whole representative cohort.

For comparing a measured profile to a reference profile, the different components of said profiles are advantageously weighted, using any appropriate statistical method which provides for greater statistical power than independent comparison of each of said components.

According to a preferred embodiment of the method according to the invention, with only one reference profile, the method comprises the following steps:

> (i) the expression levels of $n$ ($n \geq 2$) genes are measured in a biological sample from said patient, wherein at least two of these genes are selected in the group consisting of HLA-DRB4, FOSB, GPR109B, RBP7, TLR7, IL15, HLA-C, AMFR, LOC96610, IRF5, MGC29506, EDG3, IGLV1-44, IFIT2, IFI44, EPSTI1, TNFRSF17, AREG, THBS1, CTLS1, TFPI, PHACTR2, PFKFB2 and CHIT 1;
> (ii) a score is calculated as follows:

$$Score = \hat{y} = \sum_{j=1}^{n} \hat{\beta}_j \widetilde{X}_j \qquad (1)$$

> wherein $\widetilde{X}_j$ ($j = 1$ to $n$) are the expression levels of said genes measured in said biological sample, preferably expressed as centered and reduced data, and $\hat{\beta}_j$ ($j = 1$ to $n$) are calculated regression coefficients;
> (iii) the score obtained in step (ii) is interpreted by comparing it to a predetermined threshold.

In the above method, the regression coefficients ($\beta_j$) can be calculated by any multivariate method, such as PLS-R or SVM, from data collected from a representative cohort. The threshold is determined by the skilled artisan, from the same representative cohort, and depending on the wished sensitivity and specificity. Two examples of such equations, with associated threshold and tables of contingency (referring to the tested cohort), are disclosed in the experimental part below.

In a preferred embodiment of the above method, one, two or three of the genes selected in step (i) is/are selected in the group consisting of HLA-DRB4, AREG and FOSB. According to this embodiment, additional genes can be selected, without limitation, amongst GPR109B, RBP7, TLR7, THBS1 and CTLS1. For example, the set of genes selected in step (i) can comprise $n$ genes, with $n \geq 8$, among which are HLA-DRB4, AREG, FOSB, GPR109B, RBP7, TLR7, THBS1 and CTLS1 genes.

According to another preferred embodiment of this method, the set of genes selected in step (i) comprises HLA-DRB4, FOSB, GPR109B, RBP7, TLR7, IL15, HLA-C, AMFR, LOC96610, IRF5, MGC29506, EDG3, IGLV1-44, IFIT2, IFI44, EPSTI1, TNFRSF17, AREG, THBS1, CTLS1, TFPI, PHACTR2, PFKFB2 and CHIT1 genes, it being understood that other genes not cited in this list can be added to said set of genes. Indeed, the genes cited in this list have been identified by using a micro-array technique which presents certain limitations, and identification of additional relevant genes by another technique must be envisioned.

The methods described above can advantageously used for establishing a prognosis for a patient in septic shock with at least one additional organ failure. By using these methods, a physician has an early evaluation of the mortality risk of the patient in severe sepsis or septic shock, within a few hours after the onset of a second organ failure (or after admission of said patient, if the patient already has two organ failures upon admission).

According to a particular embodiment of said methods, the biological sample comprises white blood cells after removal of mature granulocytes. Alternatively, biological samples as whole blood or peripheral blood mononuclear cells (PBMC) can be used to perform the above methods.

From the HLA-DRB4 status and/or the above score $\hat{y}$, the invention helps the physician to quickly decide the pharmacological treatment to be administered and is part of the present invention. For example, the above method can be used to determine if a given patient will be a good candidate for Activated Protein C (APC also called drotrecogin alpha). According to this particular embodiment, the absence of HLA-DRB4 expression or a score $\hat{y}$ <0.03 indicates the poor prognosis risk and that administration of APC to said patient is appropriate.

Another aspect of the present invention is a method for determining if a subject in severe sepsis with at least two organ failures can benefit from the administration of a given medicinal product. Indeed, the inventors have noticed that depending on the expression profile at day 0, administration of certain costly molecules can be necessary or, to the contrary, can be more risky than beneficial. In particular, HLA-DRB4 status in said patient seems to be an important criterion to be considered. Accordingly, the present invention concerns a method for determining if a subject in severe sepsis with at least two organ failures can benefit from the administration of a given medicinal product, comprising a step of *in vitro* determining if said patient expresses HLA-DRB4.

According to a particular aspect of the above method, the absence of HLA-DRB4 in a patient expression indicates that administration of Activated Protein C (APC, also called drotrecogin alpha) to said patient is appropriate.

Alternatively or complementarily, the expression level of AREG and/or FOSB genes in said patient, or to other genes cited above, can be measured to help the physician to quickly decide the pharmacological treatment to be administered to said patient. For example, in the absence of HLA-DRB4 expression, up-regulation of one or several genes selected amongst FOSB, GPR109B, RBP7, TLR7, IL15, HLA-C, AMFR, LOC96610, IRF5, MGC29506, EDG3, IGLV1-44, IFIT2, IFI44, EPSTI1 and TNFRSF17, and/or down-regulation of one or several genes selected amongst AREG, THBS1, CTLS1, TFPI, PHACTR2, PFKFB2 and CHIT1 indicate(s) that said patient will be a good responder to Activated Protein C.

In a particular embodiment, a score can be calculated for said patient, as described above, and the physician will consider HLA-DRB4 status and/or said score to determine if said patient is in need of a particular treatment (such as Activated Protein C).

Another aspect of the present invention concerns the evaluation of the efficiency of an administered high tech treatment, which comprises a step of measuring the expression level of one or several genes selected in the group. When performing this method, the expression level will preferably be measured one, two and/or three days after the beginning of said high tech treatment.

Another aspect of the present invention concerns the evaluation of the efficiency of a (new high tech) treatment. This treatment may induce per se different modifications of gene expression between the responder and the non-responder. Indeed, the inventors have observed that when a patient is a good responder to a particular treatment, a normalization of the level of expression of the marker genes cited above can be observed in the hours and/or days following the beginning of the treatment. Accordingly, the present invention pertains to a method for evaluating the efficiency of a treatment which has been given to a patient in severe sepsis with at least two organ failures and, in particular, to a patient in septic shock with at least one additional organ failure, wherein said method comprises a step of measuring the expression level of one, two or more genes selected in the group consisting of HLA-DRB4, FOSB, GPR109B, RBP7, TLR7, IL15, HLA-C, AMFR, LOC96610, IRF5, MGC29506, EDG3, IGLV1-44, IFIT2, IFI44, EPSTI1, TNFRSF17, AREG, THBS1, CTLS1, TFPI, PHACTR2, PFKFB2 and CHIT1 in said patient before the beginning of said pharmaceutical treatment, and one, two, three or more times after the beginning of said pharmaceutical treatment. When performing this method, the expression level will preferably be measured one, two and/or three days after the beginning of said pharmaceutical treatment. Optionally, a score $\hat{y}$ is calculated as described above, for each time point. The obtained expression profiles (or score) will then be compared to each other.

According to a particular embodiment of the above method, an up-regulation of one or several genes selected amongst FOSB, GPR109B, RBP7, TLR7, IL15, HLA-C, AMFR, LOC96610, IRF5, MGC29506, EDG3, IGLV1-44, IFIT2, IFI44, EPSTI1 and TNFRSF17, and/or a down-regulation of one or several genes selected amongst AREG, THBS1, CTLS1, TFPI, PHACTR2, PFKFB2 and CHIT1 following the beginning of the pharmaceutical treatment indicate(s) that said treatment has been beneficial to the patient, it being understood that the up- or down-regulation herein corresponds to the evolution of the expression level of a given gene in said patient between the first measure at D0 and the measures following the administration of the pharmaceutical treatment. When a score $\hat{y}$ is calculated as described above, an increase of said score is also indicative of a good response to the treatment by said patient.

The present invention also relates to a method for selecting patients to be enrolled in a clinical trial for evaluating a medicinal product in the treatment of severe sepsis, especially in the treatment of septic shock. Indeed, the future development of new drugs and the performance of clinical trials would be helped if only the patients with a poor prognosis are included, thereby eliminating the "noise" coming from patients who would have recovered without the drug under examination. A method for selecting subjects to be enrolled in a clinical trial for evaluating a medicinal product in the treatment of severe sepsis with at least two organ failures, comprising a step of *in vitro* establishing a prognosis for said subjects, by any of the methods described above, is hence also part of the present invention. According to this method, the subjects enrolled in the clinical trial are preferably those who have a poor prognosis, i.e., when a representative cohort is used to define references, those who have an expression profile at day 0 (for a set of genes as defined above) which is more similar to that of subjects who have died from severe sepsis than to the profile of the group of subjects who survived. When a score $\hat{y}$ is calculated as described above, the subjects to be enrolled in the clinical trial are those who have a score inferior to the predetermined threshold. Alternatively the skilled artisan can chose to enrol subjects only upon determination of their HLA-DRB4 status, since this seems to be of a critical relevance.

When performing the methods according to the invention, the level of expression of the selected genes can be measured by any technique, either at the mRNA level, or at the protein level. In a preferred embodiment of the methods according to the invention, the transcription level of these genes is measured. The skilled artisan knows several techniques to perform such a measure, and will use the technique which is the most convenient having regard to the context. Particular parameters to consider are the rapidity of the result, its reliability, and the cost of the measure. For example, the level of mRNA encoding the genes of interest can be measured by real-time RT-PCR. A detailed protocol for doing so for HLA-DRB4, including the primers sequence, is described in the experimental part below, but the skilled artisan can perfectly modify this protocol. Alternatively, the level of mRNA encoding the selected genes can be measured by macro- or micro-array, using either a standard chip or a chip which has been designed specifically for performing the methods of the present invention. In a particular embodiment of the above methods, the expression level of one or several genes selected in the group consisting of HLA-DRB4, FOSB, GPR109B, RBP7, TLR7, IL15, HLA-C, AMFR, LOC96610, IRF5, MGC29506, EDG3, IGLV1-44, IFIT2, IFI44, EPSTI1, TNFRSF17, AREG, THBS1, CTLS1, TFPI, PHACTR2, PFKFB2 and CHIT1 is measured by microarray.

As described in the experimental part below, the inventors have observed that when the HLA-DRB4 gene is present in the genome of a patient, this gene is always expressed. The above-described methods can hence comprise a step of HLA-DR genotyping. The HLA-DR loci comprise only one alpha gene and 4 functional beta genes called DRB1, DRB3, DRB4 and DRB5. More than 100 different alleles encode DRB1, which have been grouped into 13 different haplotypes DR1-DR16, while there is little allelic diversity for DRB3, 4 and 5. It is now established that particular DRB1 alleles are always associated with DRB4 (Bodmer et al., 1992; Nepom and Erlich, 1991). Indeed, the presence in DRB1 of a DR4, DR7 or DR9 allele implies that the DRB4 gene is present in the genome. As a consequence, the methods according to the present invention can comprise a step of genotyping either the DRB1 locus, or the DRB4 locus (or both). Of course, such a genotyping step can be done as an alternative or in addition to the determination of the transcription level of the HLA-DRB4 (putative) gene. Any technique known by the skilled artisan can be used to perform this genotyping, such as, for example, a semi-automated method as described by Pachot *et al*. (Pachot et al., 2007).

The methods described above can also comprise, as an alternative or in addition, a step of measuring, in a sample from said patient, the level of one or several of the proteins encoded by the genes cited in the above list. This can be done by any immunoassay which the skilled artisan will consider as appropriate.

Another important aspect of the present invention is a kit for performing any of the methods described above. In a particular embodiment, a kit according to the invention comprises one, two or three pairs of primers, wherein each pair of primers is specific for a gene selected amongst HLA-DRB4, AREG and FOSB. Such a kit can also comprise probes for performing real-time amplification of part of one or several of the selected genes. A set of primers and probe for real-time amplification of part of the HLA-DRB4 gene is described in the experimental part below. Of course, the skilled artisan can chose to use different primers and probes specific for HLA-DRB4 to constitute a kit according to the invention, and the sequences indicated herein are not limitative. In another embodiment of the kit of to the invention, the kit is adapted for genotyping the HLA-DRB4 gene. According to this embodiment, the kit can comprise, for example, reagents for DNA extraction. A kit according to the invention will also advantageously comprise a notice mentioning the relevance of the level of expression of the selected genes in the prediction of the outcome of severe sepsis with at least two organ failures.

Of course, a kit according to the present invention can further comprise one or several additional pairs of primers, wherein each pair of primers is specific for one sequence selected in the group consisting of 18S rRNA, GPR109B, RBP7, TLR7, IL15, HLA-C, AMFR, LOC96610, IRF5, MGC29506, EDG3, IGLV1-44, IFIT2, IFI44, EPSTI1, TNFRSF17, THBS1, CTLS1, TFPI, PHACTR2, PFKFB2 and CHIT1 genes.

In the above kits, the primer(s) and/or probe(s) can advantageously be labeled. Any labeling technique known by the skilled artisan can be used to that purpose.

Reagents and/or enzymes for performing amplification reactions can also be included in such a kit. The skilled artisan perfectly knows which reagents and enzymes can be used (and therefore, introduced in the kits) to perform amplification reactions such as polymerase chain reaction (PCR), reverse transcription-polymerase chain reaction (RT-PCR) and the like.

In another embodiment of a kit according to the invention, this kit comprises a chip enabling hybridization of nucleic acids specific for at least two genes selected in the group consisting of HLA-DRB4, AREG, FOSB, GPR109B, RBP7, TLR7, IL15, HLA-C, AMFR, LOC96610, IRF5, MGC29506, EDG3, IGLV1-44, IFIT2, IFI44, EPSTI1, TNFRSF17, THUS1, CTLS1, TFPI, PHACTR2, PFKFB2 and CHIT1.

Such a kit will preferably also comprise a notice of use, which will advantageously explain how to interpret the results.

The kits according to the present invention can be designed to perform the above-described methods either from a whole blood sample, or from isolated PBMC. In this latter case, the kits can optionally also comprise reagents for PBMC isolation.

Other characteristics of the invention will also become apparent in the course of the description which follows of the biological assays which have been performed in the framework of the invention and which provide it with the required experimental support, without limiting its scope.

## EXAMPLES

**Material and Methods**

*Patients*

The multicenter study was approved by the Cochin Hospital Ethics Committee (# CCPPRB 2061) and concerned patients from 4 Intensive Care Units in France (2 medical and 2 surgical ICUs from AP-HParis), having septic shock defined according to the ACCP/SCCM consensus. The patients have been enrolled after obtaining written informed consent from their next of kin. Inclusion criteria: patients in septic shock having at least two organ failures (defined by sequential organ failure assessment (Vincent et al., 1998) related to sepsis, within the 24 hours after the occurrence of the second organ failure; exclusion criteria: age lower than 18 y/o; treatment for cancer within the last 6 months; recent treatment for immune or hematologic diseases; a life expectancy inferior to 6 months. The day of inclusion corresponded to the first blood sampling quoted day 0, within 24 hours after the occurrence of the second organ failure. Blood samples consisted in 15 ml on EDTA.

Sixteen patients died within the 28-day follow-up, although 50% were treated by APC. Among the twelve patients (over 48) treated with APC, 8 patients (67%) died.

*Total RNA isolation*

White cells were isolated by gradient centrifugation (Histopaque, Sigma, St Quentin Fallavier, France) to eliminate mature polymorphonuclear cells. Total RNA was extracted using the Qiagen Rneasy kit (Qiagen GmbH, Germany). All RNA samples were treated with RNase-free DNAse.

*Microarrays procedure*

Quality of RNA samples was assessed using RNA 6000 Nano chips (Agilent technologies, Palo Alto, CA, USA) and quantity of RNA samples was assessed by measuring the absorbance at 260 nm with spectrophotometer NanoDrop ND-1000 (Labtech international Biotech, Ringmer, UK). Preparation of cRNA was performed according to the protocols of the manufacturer (Affymetrix, Santa Clara, CA USA). Briefly, 5µg of RNA samples were used to generate first-strand cDNA using a T7-oligo(DT) primer and the Superscript II Reverse Transcriptase. Second-strand synthesis was achieved using a cocktail of enzymes from E. coli (DNA ligase and DNA polymerase I), Rnase H followed by an incubation with T4 DNA polymerase. Clean-up of double-stranded cDNA was done using the GeneChip Sample Cleanup Module (Affymetrix). *In vitro* transcription was carried out in the presence of T7 RNA Polymerase and a biotinylated nucleotide analog/ribonucleotide mix for complementary RNA (cRNA) amplification and biotin labelling (GeneChip IVT Labeling Kit). Resulting biotinylated cRNA were cleaned-up using the GeneChip Sample Cleanup Module and quantified by absorbance measurement at 260 nm (NanoDrop). Starting with 5µg of total RNA yielded between 50 and 80µg of purified cRNA. Then, 20 µg of cRNA from samples were incubated at 94˚C for 35 min in a fragmentation buffer to be reduced into a mean size of approximately 35-200 nucleotides and finally added to the hybridisation buffer. Fragmented cRNA were hybridised on Affymetrix HG-U133 Plus 2.0 array for 16 hours at 45˚C together with internal hybridisation controls (bioB, bioC, bioD, cre and oligonucleotide B2). The washing and staining procedure was performed in the Affymetrix Fluidics Station 450. Probe arrays were exposed to 10 washes in non-stringent wash buffer A (6x SSPE, 0.01% Tween20) at 30˚C, followed by 6 washes in stringent buffer B (100mM MES, 0.1M [Na$^+$], 0.01% Tween20) at 50˚C. The biotinylated cRNA were stained with a streptavidin-phycoerythrin conjugate (SAPE, 10µg/ml) 5 minutes at 35˚C and washed again with non-stringent buffer A (10 washes at 30˚C). An antibody amplification step was added using goat IgG (0.1mg/ml) and anti-streptavidin antibody biotinylated (3µg/ml) followed by an additional SAPE stain (5 minutes at 35˚C for each step). Finally, arrays were washed 15 times in non-stringent buffer A at 35˚C before scanning in Affymetrix GeneChip Scanner 3000.

The array contains ~54600 human probe sets corresponding to approximately 22400 Unigene clusters. CEL files were produced using GCOS (GeneChip® Operating Software). Normalisation of data was performed using GC-robust multi-array average (GC-RMA) that generates intra- and inter-chips normalizations in a single step.

***Real time PCR***

To validate the levels of expression of genes obtained by microarray analysis, a real time PCR was performed for the 8 most important genes and for an endogenous control eukaryotic 18S rRNA. Reverse transcription reactions were performed according to established methods or manufacturer specifications (High Capacity cDNA Archive kit, Applied Biosystems, Foster City, CA, USA). Briefly, the probes contain a 6-carboxy-fluorescein phosphoramidite (FAM dye) label at the 5' end of the gene and a minor groove binder and nonfluorescent quencher at the 3' end and are designed to hybridize across exon junctions. The assays are supplied with primers and probe concentrations of 900 nM and 250 nM, respectively.

The gene expression assay for HLA-DRB4 was performed with the following primers and probe, which have been used at the same concentration for the three of them:

Forward primer: TTTGTGCTTCCCTTTACCTAAACTG (SEQ ID No: 1);
Reverse primer: AATAATGCAATGTGTGGCACAAG (SEQ ID No: 2); and
Probe: CCTGCCTCCCGTGCATCTGTACTCC (SEQ ID No: 3).

The other selected assays were referenced as "inventoried" assays (inventoried Assay ID, Applied Biosystems). Information about these assays are provided in Tables 1 and 2 below.

Table 1: References of the gene expression assays designed and distributed by Applied Biosystems and used in the present study.

| Gene Symbol | Gene Name | NCBI Gene ref | Asay ID |
|---|---|---|---|
| HLA- DRB4 | major histocompatibility complex, class II, DR beta 4 | NM_021983 | - |
| FOSB | FBJ murine osteosarcoma viral oncogene homolog B | NM_006732.1 | Hs00171851_m1 |
| AREG | amphiregulin (schwannoma-derived growth factor) | NM_001657.2 H | s00155832_m1 |
| GPR109B | G protein-coupled receptor 109B | NM_006018.1 | Hs02341102_s1 |

(continued)

| Gene Symbol | Gene Name | NCBI Gene ref | Asay ID |
|---|---|---|---|
| RBP7 | retinol binding protein 7, cellular | NM_052960.1 | Hs00364812_m1 |
| THBS1 | thrombospondin 1 | NM_003246.2 | Hs00962914_m1 |
| TLR7 | toll-like receptor 7 | NM_016562.3 | Hs00152971_m1 |
| CTSL1 | cathepsin L1 | NM_145918.2 | Hs00377632_m1 |
| 18S | Eukaryotic 185 rRNA | X03205.1 | Hs99999901_s1 |

Table 2: Nucleotide sequence surrounding each probe designed by Applied Biosystems

| Gene | Chromosome | Target exon | Context sequence | SEQ ID No: |
|---|---|---|---|---|
| FOSB | 19 | 1 | CGGCCTCCCAGGAGTGCGCCGGTCT | 4 |
| AREG | 4 | 4 | CAGTCCAGCTTAGAAGACAATACGT | 5 |
| GPR109B | 12 | 6 | GAAGAAGAAGTTGCTGATCCAGAAT | 6 |
| RBP7 | 1 | 3 | ACTCCACCTGGAAATGTTCTGTGAA | 7 |
| THBS1 | 15 | 21 | AAATACGAATGTAGAGATCCCTAAT | 8 |
| TLR7 | X | 2 | AAAAATGGTGTTTCCAATGTGGACA | 9 |
| CTSL1 | 9 | 7 | GAAGAACAGCTGGGGTGAAGAATGG | 10 |
| 18S | | | TCCATTGGAGGGCAAGTCTGGTGCC | 11 |

To control possible variations among PCR runs, which have been performed on different days, 2 types of negative PCR controls were done (PCR substrate resulting of a RT without template = no template control (NTC) for each plate of amplification and for each TaqMan Gene Expression Assay. Absence of contamination of the PCR mix was checked by looking at PCR substrate resulting from RT without polymerase (= No amplification control, NAC), which had to generate no fluorescence signal. Eukaryotic 18S ribosomal RNA was used as the endogenous RNA controls (Assay ID: Hs99999901_s1; Applied Biosystems).

The level of messenger RNA (mRNA) was measured at day 0 with TaqMan Gene Expression Assays on Rotorgene 3000 (Corbett Life Sciences, Sydney). Genes were selected because of their differential expression on the microarray between dead and alive patients.

## *Genotyping*

Because of the large difference in HLA-DRB4 expression between survivals and dead patients (FC>5), the genotyping of this gene was performed in all patients. Genomic DNA used for HLA-DRB4 typing was extracted through salting-out technique from fresh peripheral blood leucocytes. HLA-DRB4 samples were typed at high resolution DNA based typing (allelic level) using the Polymerase Chain Reaction-Sequence Specific Primers (PCR-SSP) amplifications (one Lambda, Inc., Canoga Park, CA or Genovision).

## Statistics

*Standard statistical analysis*: quantitative results were expressed in median $\pm$ Inter Quartile Range (IQR). Comparison of patient clinical characteristics was done for quantitative measures with a Mann-Whitney test and for qualitative measures with Fisher exact test at day 0. $p < 0.05$ was considered as statistically significant.

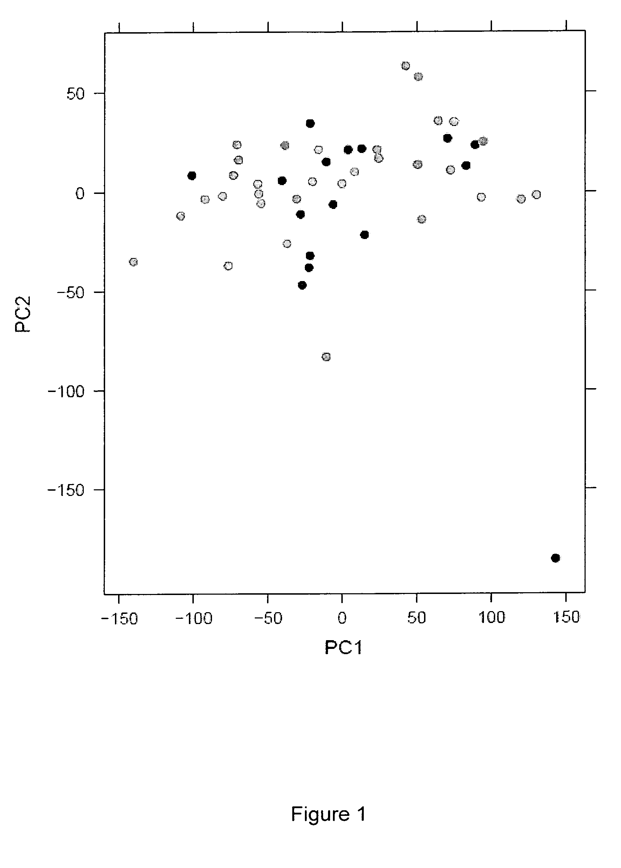

*Microarray analysis*: genes with significant differential expression between surviving and non-surviving patients were identified using statistical packages from Bioconductor (http://www.bioconductor.org). After GC-RMA (*Gene Chip Robust Multiarray Averaging*) normalization (Irizarry et al., 2003), data were subjected to unpaired t-test for outcome at D0. Global transcriptional profiles were visualized in 2-dimensional space spanned by the two first factors of the Principal Component Analysis (PCA) at day 0 (figure 1). Twenty-nine sets of probes, corresponding to 24 genes, were identified at day 0 (tables 3 and 4) and were analyzed using hierarchical clustering methods. Among them, 8 genes were further selected as a top list (table 2), taking into account the classification by p-value and fold change (FC).

Table 3: list of sets of probes which were differentially expressed and selected according to outcome at D0 (alive versus dead). DR means downregulated, and UR upregulated. Classification considering p-value

| Probe Set ID | Gene Symbol | Gene title | DF | p-value | REG |
|---|---|---|---|---|---|
| 205239_at | **AREG** | amphiregulin (schwannoma-derived growth factor) /// similar to | -3,15 | 1,90E-04 | DR |
| 202768_at | **FOSB** | FBJ murine osteosarcoma viral oncogene homolog B | 3,97 | 2,53E-03 | UR |
| 201110_s_at | **THBS1** | thrombospondin 1 | -2,41 | 5,13E-03 | DR |
| 220146_at | **TLR7** | toll-like receptor 7 | 2,28 | 7,94E-03 | UR |
| 238066_at | **RBP7** | retinol binding protein 7, cellular | 2,44 | 1,30E-02 | UR |
| 202087_s_at | **CTSL** | cathepsin L | -2,08 | 1,60E-02 | DR |
| 210664_s_at | TFPI | tissue factor pathway inhibitor (lipoprotein-associated coagulant | -2,03 | 2,02E-02 | DR |
| 205992_s_at | IL15 | interleukin 15 | 2,20 | 2,60E-02 | UR |
| 214768_x_at | HLA-C | Major histocompatibility complex, class I, C | 2,65 | 3,16E-02 | UR |
| 201109_s_at | THBS1 | Thronbospondin 1 | -2,16 | 3,23E-02 | DR |
| 202203_s_at | AMFR | autocrine motility factor receptor | 2,01 | 3,31 E-02 | UR |
| 224342_x_at | LOC96610 | Hypothetical protein similar to KIAA0187 gene product ///Hyp | 2,20 | 3,47E-02 | UR |
| 239412_at | IRF5 | interferon regulatory factor 5 | 2,23 | 3,91 E-02 | UR |
| 223565_at | MGC29506 | hypothetical protein MGC29506 | 2,23 | 4,19E-02 | UR |
| 213258 at | TFPI | tissue factor pathway inhibitor (lipoprotein-associated coagulant | -2,05 | 4,22E-02 | DR |

(continued)

| Probe Set ID | Gene Symbol | Gene title | DF | p-value | REG |
|---|---|---|---|---|---|
| 221286_s_at | MGC29506 | hypothetical protein MGC29506 | 2,05 | 4,33E-02 | UR |
| 244774_at | PHACTR2 | Phosphatase and actin regulator 2 | -2,07 | 4,36E-02 | DR |
| 226733_at | PFKFB2 | 6-phosphofructo-2-kinase/ fructose-2,6 biphosphatase 2 | -2,21 | 4,61E-02 | DR |
| 205220_at | **GPR109B** | Gprotein-coupled receptor 109B /// G protein-coupled receptor | 2,79 | 4,92E-02 | UR |
| 209728_at | **HLA-DRB4** | major histocompatibility complex, class II, DR beta 4/// major | 5.18 | 5,29E-02 | UR |
| 208168_s_at | CHIT1 | chitinase 1 (chitotriosidase) | -2,55 | 5,52E-02 | DR |
| 215775_at | THBS1 | thrombospondin 1 | -2,07 | 5,67E-02 | DR |
| 217179_x_at | LOC96610 | Hypothetical protein similar to KIAA0187 gene product | 2,18 | 6,09E-02 | UR |
| 228176 at | EDG3 | endothelial differentiation, sphingolipid G-portein-coupled rece | 2,18 | 6,63E-02 | UR |
| 234764 x at | LOC96610 /// | Hypothetical protein similar to KIAA018 gene product/// Imm | 2,03 | 7,48E-02 | UR |
| 217502 at | IFIT2 | interferon-induced protein with tetratricopeptide repeats 2 | 2,15 | 7,78E-02 | UR |
| 214453 s at | IF144 | interferon-induced protein 44 | 2,07 | 7,96E-02 | UR |
| 227609 at | EPSTI1 | epithelial stromal interaction 1 (breast) | 2,44 | 8,28E-02 | UR |

(continued)

| Probe Set ID | Gene Symbol | Gene title | DF | p-value | REG |
|---|---|---|---|---|---|
| 206641 at | TNFRSF17 | tumor necrosis factor superfamily, member 17 | 2,45 | 9,47E-02 | UR |

Table 4: list of sets of probes which were differentially expressed and selected according to outcome at D0 (alive versus dead). DR means downregulated, and UR upregulated. Classification considering fold change (DF)

| Probe Set ID | Gene Symbol | Gene title | DF | p-value | REG |
|---|---|---|---|---|---|
| 209728 at | **HLA-DRB4** | major histocompatibility complex, class II, DR beta 4/// major | 5,18 | 5,29E-02 | UR |
| 202768_at | **FOSB** | FBJ murine osteosarcoma viral oncogene homolog B | 3,97 | 2,53E-03 | UR |
| 205239 at | **AREG** | amphiregulin (schwannoma-derived growth factor) /// similar to | -3,15 | 1,90E-04 | DR |
| 205220_at | **GPR109B** | G protein-coupled receptor 109B /// G protein-coupled receptor | 2,79 | 4,92E-02 | UR |
| 214768 x at | HLA-C | Major histocompatibility complex, class I, C | 2,65 | 3,16E-02 | UR |
| 208168_s_at | CHIT1 | chitinase 1 (chitotriosidase) | -2,55 | 5,52E-02 | DR |
| 206641 at | TNFRSF17 | tumor necrosis factor receptor superfamily, member 17 | 2,45 | 9,47E-02 | UR |
| 227609 at | EPSTI1 | epithelial stromal interaction 1 (breast) | 2,44 | 8,28E-02 | UR |
| 238066_at | **RBP7** | retinol binding protein 7, cellular | 2,44 | 1,30E-02 | UR |
| 201110_s_at | **THBS1** | thrombospondin 1 | -2,41 | 5,13E-03 | DR |
| 220146 at | **TLR7** | toll-like receptor 7 | 2,28 | 7,94E-03 | UR |

(continued)

| Probe Set ID | Gene Symbol | Gene title | DF | p-value | REG |
|---|---|---|---|---|---|
| 239412 at | IRF5 | interferon regulatory factor 5 | 2,23 | 3,91E-02 | UR |
| 223565 at | MGC29506 | hypothetical protein MGC29506 | 2,23 | 4,19E-02 | UR |
| 226733 at | PFKFB2 | 6-phosphofructo-2-kinase/ fructose-2,6 biphosphatase 2 | -2,21 | 4,61E-02 | DR |
| 224342 x at | LOC96610 | Hypothetical protein similar to KIAA0187 gene product *III/Hypo* | 2,20 | 3,47E-02 | UR |
| 205992 s at | IL15 | interleukin 15 | 2,20 | 2,60E-02 | UR |
| 217179_x_at | LOC96610 | Hypothetical protein similar to KIAA0187 gene product | 2,18 | 6,09E-02 | UR |
| 228176 at | EDG3 | endothelial differentiation, sphingolipid G-portein-coupled rece | 2,18 | 6,63E-02 | UR |
| 201109-s-at | THBS1 | Thrombospondin 1 | -2,16 | 3,23E-02 | DR |
| 217502 at | IFIT2 | interferon-induced protein with tetratricopeptide repeats 2 | 2,15 | 7,78E-02 | UR |
| 202087_s_at | **CTSL** | cathepsin L | -2,08 | 1,60E-02 | DR |
| 244774 at | PHACTR2 | Phosphatase and actin regulator 2 | -2,07 | 4,36E-02 | DR |
| 215775_at | THBS1 | thrombospondin 1 | -2,07 | 5,67E-02 | DR |
| 214453_ s_at | IFI44 | interferon-induced protein 44 | 2,07 | 7,96E-02 | UR |
| 213258_ at | TFPI | tissue factor pathway inhibitor (lipoprotein-associated coagulant | -2,05 | 4,22E-02 | DR |
| 221286_s_at | MGC29506 | hypothetical protein MGC29506 | 2,05 | 4,33E-02 | UR |

(continued)

| Probe Set ID | Gene Symbol | Gene title | DF | p-value | REG |
|---|---|---|---|---|---|
| 210664_s_at | TFPI | tissue factor pathway inhibitor (lipoprotein-associated coagulant | -2,03 | 2,02E-02 | DR |
| 234764_x_at | LOC96610 /// | Hypthetical protein similar to KIAA0187 gene product ///Imm | 2,03 | 7,48E-02 | UR |
| 202203_s_at | AMFR | autocrine motility factor receptor | 2,01 | 3,31E-02 | UR |

### Correlations for validation of microarray with PCR

Linear correlation between the Ct ($Ct_{gene}$ - $Ct_{18S}$) and logarithm base 2 of the fluorescence intensity ($\log_2$int) was studied by the Spearman correlation test. $p<0.05$ was considered as statistically significant.

### Building of a predictive model

Predictive models can be built by using any multivariate method known by the skilled artisan. In the present case, Partial Least Squares Regression has been used, but other methods, such as Support Vector Machines, can also be used to that purpose. The principles of these methods are recalled below.

*Multivariate methods: introduction*

Denote by $X = (X_1,...,X_p$ a $p$-dimensional space of variables representing the set of predictors and by y the response variable. Throughout this section, and without loss of generality, a centered version of $X$ and $y$ are considered. Linear model seeks to associate $X$ to the response $y$ with respect to the following theoretical model:

$$y = X\beta + \varepsilon$$

Numerous methods aim at estimating the regression coefficients β; Partial Least Squares Regression (PLS-R) and the Support Vector Machines (SVM), which are among the most efficient state-of-the-art approaches for classification purposes (Rosipal et al., 2003; Tenenhaus et al., 2007), are further described below. If $\hat{\beta}^{PLS}$ and $\hat{\beta}^{SVM}$ are the PLS-R and SVM estimator, respectively:

$$\hat{y}^{PLS} = X\hat{\beta}^{PLS} \ \text{ and } \ \hat{y}^{SVM} = X\hat{\beta}^{SVM}$$

*Partial Least Squares Regression*

The main idea of PLS-R (Wold et al.; 1983; Tenenhaus et al., 1998) is to extract from $X$ a set of mutually orthogonal components T = ($t_1$,...,$t_h$), that have a large covariance with $y$, linear combination of the original variables, which serve as new regressors to predict the response variable $y$. It turns out that to get the following final PLS-R model, it is necessary to adjust the number of PLS components. Finally, once $h$ fixed, we have:

$$\hat{y}^{PLS}(h) = P^{\perp}_{t_1,...,t_h} y = X\hat{\beta}^{PLS}(h),$$

where $P^{\perp}$ is the projection operator and $h$ the number of retained PLS components. $h$ is usually chosen by maximizing the prediction accuracy, estimated thanks to a Cross-Validation technique.

*Support Vector Machines*

Support Vector Machines (SVM) (Boser et al, 1992; Vapnik, 1998) seeks the optimal separating hyperplane (the so-called margin) that separates two classes of observations (*e.g.*, "dead patients" versus "surviving patients"). The margin is defined as the distance between the hyperplane and its nearest points. It can be shown that choosing the optimal separating hyperplane results in an improvement of the trained classifier to predict new observations (Vapnik, 1998). To find $\hat{\beta}^{SVM}$ , one needs to solve the following problem:

$$\hat{\beta}^{SVM}(\lambda) = arg\ \min_{\beta}\{max(0, 1 - y' X\beta)\} + \lambda\|\beta\|$$

Once again, it turns out that to get the following final SVM model, it is necessary to determine $\lambda$,

$$\hat{y}^{SVM}(\lambda) = X\hat{\beta}^{SVM}(\lambda)$$

This number is usually chosen by Cross-Validation.

*Implementation*

a) the R software (version 2.5.1) was used to classify the patients of the present study. For PLS-R model the "pls" packages was used and for SVM model, the "e1071" package. The references of these packages are as follows:

- Ron Wehrens and Bjørn-Helge Mevik (2007). *pls: Partial Least Squares Regression (PLSR) and Principal Component Regression (PCR).* R package version 2.0-1(http://mevik.net/work/software/pls.html).

- Evgenia Dimitriadou, Kurt Hornik, Friedrich Leisch, David Meyer and Andreas Weingessel (2006). *e1071: Misc Functions of the Department of Statistics (e1071),* TU Wien. R package version 1.5-16.

b) A bootstrapped Hold Out technique was used to estimate the prediction accuracy:

- 2/3 of the total dataset was randomly chosen to be the training set
- The parameters $h$ or $\lambda$ of the classifier were computed, for each training set based on a Leave One Out cross validation technique
- This was repeated 100 times to correctly estimate the average of the prediction accuracy.

Genes of interest have been selected on the microarray differential expression (see above) (fold change >2 and p value <0.1). The 24 genes related to 29 selected sets of probes (tables 3 and 4) correlating with outcome were grouped to build a predictive model based on Partial Least Squares (PLS) regression, useful to deal with correlated data situation encountered in the data set (as verified by the correlation matrix). The relative contribution of each gene was weighted by a coefficient and put in an equation allowing the estimation of the outcome for a new individual. A first equation including the 29 sets of probes was built (equation 2). Additionally, the inventors built an equation (equation 3) including the 8 selected genes (table 2), more adapted to available methods of detection of gene expression in nowadays clinical context. Of course, additional patient data can be included in the model and improve the accuracy of coefficients.

***Statistical strategy:***

Based on the list of genes of interest given in Tables 3 and 4, different steps were performed to determine a relevant threshold, usable for classifying a new given patient requiring prognostic evaluation at day 0 of septic shock plus 1 organ failure.

Correspondence with ID and SYMBOLS was made using the library hgu133plu2 (v. 1.16.0) from BioConductor. Data were first centered and reduced and are summarized in the table 5.

Table 5: mean and SD from a representative cohort to calculate centered and reduced data.

| SYMBOL | mean | SD |
|---|---|---|
| AREG | 4,008 | 1,453 |
| FOSB | 6,091 | 2,230 |
| THBS1 | 9,765 | 1,523 |
| TLR7 | 4,038 | 1,483 |
| RBP7 | 6,776 | 1,696 |
| CTSL1 | 8,274 | 1,450 |
| TFPI | 5,088 | 1,475 |
| IL15 | 5,793 | 1,672 |
| HLA-C | 6,802 | 2,137 |
| THBS1 | 8,228 | 1,713 |
| AMFR | 5,123 | 1,519 |
| LOC96610 | 6,058 | 1,803 |
| IRF5 | 6,502 | 1,823 |
| MGC29506 | 4,054 | 1,834 |
| TFPI | 5,129 | 1,666 |
| MGC29506 | 6,750 | 1,699 |
| PHACTR2 | 4,917 | 1,701 |
| PFKFB2 | 8,839 | 1,887 |
| GPR109B | 4,748 | 2,482 |
| HLA-DRB4 | 5,764 | 4,036 |
| CHIT1 | 6,905 | 2,310 |
| THBS1 | 4,763 | 1,820 |
| LOC96610 | 6,412 | 1,970 |
| EDG3 | 5,084 | 2,021 |
| IGLV1-44 | 7,981 | 1,891 |
| IFIT2 | 4,442 | 1,982 |
| IFI44 | 6,916 | 1,988 |
| EPSTI1 | 6,383 | 2,414 |
| TNFRSF17 | 4,853 | 2,530 |

$\widetilde{X}_1, \widetilde{X}_2, \cdots, \widetilde{X}_{29}$ correspond to the centered and reduced (or standardized) variable of the genes of interest. The score could be written as:

$$Score = \hat{y} = \sum_{j=1}^{29} \hat{\beta}_j \widetilde{X}_j$$

where $\hat{\beta}_j$ is the estimation given by the PLS regression of $y$ on $\tilde{X}_j$ $j$ = 1,2,...,29.

**Results**

### Clinical and inflammatory status of 48 patients

Results are expressed as median (interquartile range IQR). Forty eight patients of the training cohort were 66 (22 IQR) year old. At the inclusion time, they had the following characteristics: 1) a SAPSII at 59 (15 IQR) and a SOFA score at 10 (4 IQR) (Vincent et al., 1998)) 2) monocyte HLA-DR expression at 2765 (2909 IQR) AB/C and plasma IL-10 at 175 (500 IQR) pg/ml (IL-12p40 not detectable) as inflammatory parameters. Sixteen over 48 patients died within the 28-day study period. Patients who will die differed at day 0 of inclusion from alive for SAPSII (71 (21 IQR) vs 56 (16 IQR), p=0.0002) but did not differ for age, monocyte HLA-DR expression IL-10 at day 0 and sepsis related organ failure scores (SOFA).

### Microarray experiments

For 2 patients, technical problems provided non interpretable data. The non supervised transcriptome data of septic shock patients at day 0 did not allow classifying the patients according to clinical items. When patient classification was based on outcome, a set of 29 probes sets (24 gene transcripts) differed significantly between survivors and non survivors (Tables 3 and 4). HLA-DRB4 presented the highest FC (FC>5) in survivors, with a large difference of fluorescence between the group of patients expressing HLA-DRB4 (positive) and a group showing low intensities (negative). At day 0, the positive HLA-DRB4 expression corresponded to surviving patients, except for three (18% of patients with positive expression) (table 6). The negative HLA-DRB4 expression group had a high risk of death, with only 54% of patients who survived.

HLA-DRB4 expression encodes for HLA-DRB4 protein that belongs to the HLA class II gene cluster, which has been described as an important inflammatory marker in sepsis (Döcke et al., 1997; Venet et al., 2007). In our population, the over-expression of HLA-DRB4 transcript was associated with a good outcome. The very large difference in expression between dead and alive patients motivated to genotype this gene.

Interestingly, it was mostly the group of negative expression of HLA-DR B4 which received APC treatment (8 over 12 treated patients), decided by the physician in charge who ignored the result of genotyping.

| Patients | DRB4 status | Microarray (logGCRMA) | Microarray (rawGCRMA) | RTqPCR (DCt) | genotyping | | | outcome | |
|---|---|---|---|---|---|---|---|---|---|
| | | | | | HLA-DR B1 (1) | HLA-DR B1 (2) | HLA-DR B4 | | |
| BEA 1003 | - | 8,67 | 407 | nd | 102 | 08 | * | alive | |
| BEJ 1023 | - | 2,53 | 6 | nd | 01 | 13 | * | dead | |
| CHJ 3095 | - | 2,29 | 5 | nd | 01 | 1001 | * | dead | |
| COP 1020 | - | 2,74 | 7 | nd | 01 | 03 | * | alive | |
| GIC 3091 | - | 2,18 | 5 | nd | 08 | 03 | * | dead | |
| HAA 1016 | - | 2,20 | 5 | nd | 13 | 13 | * | alive | |
| LAMI | - | 2,30 | 5 | nd | * | * | * | alive | |
| LEJ 4012 | - | 3,01 | 8 | nd | * | * | * | dead | |
| LOJ 1030 | - | 2,19 | 5 | 25.4 | * | * | * | dead | |
| MIM 1012 | - | 2,89 | 7 | * | 03 | 11 | * | dead | |
| RIG 4018 | - | 2,23 | 5 | nd | 11 | 16 | * | dead | 46% dead 54% alive |
| SAA 3100 | - | 2,72 | 7 | nd | 11 | 14 | * | dead | |
| SAJ 4014 | - | 2,45 | 5 | nd | 13 | 13 | * | dead | |
| STA 3096 | - | 2,24 | 5 | nd | 13 | 14 | * | dead | |
| AKY | - | 2,40 | 5 | nd | 15 | 15 | * | alive | |
| AMST | - | 2,25 | 5 | nd | 0102 | 14 | * | alive | |
| CHNO | - | 2,24 | 5 | nd | 03 | 15 | * | alive | |
| FENE | - | 2,23 | 5 | nd | 03 | 15 | * | alive | |
| FLOR | - | 2,52 | 6 | nd | * | * | * | alive | |
| DEPA | - | 2,22 | 5 | nd | 11 | 13 | * | dead | |
| CEP | - | 2,13 | 4 | nd | 11 | 13 | * | dead | |
| DACO | - | 2,10 | 4 | nd | 13 | 13 | * | alive | |
| DUJ | - | 2,76 | 7 | nd | 11 | 15 | * | alive | |
| FOE | - | 2,22 | 5 | nd | * | * | * | alive | |
| PIGS | - | 2,77 | 7 | nd | 01 | 16 | * | alive | |
| RICJ | - | 2,20 | 5 | nd | 13 | 14 | * | alive | |
| GAM 5066 | + | 3,16 | 9 | 20.3 | 07 | 08 | 01030102N | alive | |
| ABG 4013 | + | 9,91 | 959 | 17.1 | 04 | 11 | 0103 | alive | |
| BLD 1024 | + | 11,47 | 2838 | 15.7 | 07 | 12 | 0101 | alive | |
| CAC 4009 | + | 9,70 | 833 | 17.1 | 08 | 09 | 0103 | alive | |
| CEF 3094 | + | 12,83 | 7300 | 14.9 | * | * | * | alive | |
| CRJ 5011 | + | 9,90 | 955 | 17.1 | 04 | 13 | 0103 | alive | |
| DAN 1025 | + | 9,12 | 556 | 17.9 | * | * | * | alive | |
| DEF 1004 | + | 2,40 | 5 | 18.6 | 03 | 04 | 0103 | alive | |
| DEJ 1015 | + | 11,05 | 2114 | * | 04 | 11 | 0103 | alive | |
| FOA 1026 | + | 10,89 | 1894 | 16.6 | 07 | 13 | 0101 | dead | 18% dead 82% alive |
| GAJ 3102 | + | 11,28 | 2484 | 16.6 | 03 | 04 | 0103 | alive | |
| HAD 1032 | + | 6,11 | 69 | 20.2 | 04 | 11 | 0103 | alive | |
| LOJ 1027 | + | 11,43 | 2753 | 16.9 | 07 | 13 | 0101 | alive | |
| MAA 1021 | + | 9,24 | 603 | 18.2 | * | * | * | alive | |
| MEA 3101 | + | 10,78 | 1759 | 17.3 | * | * | * | alive | |
| PRL 1002 | + | 11,12 | 2219 | 16.2 | 07 | 08 | 0101 | alive | |
| TRB 1028 | + | 4,37 | 21 | 19.5 | 01 | 07 | 0103 | dead | |
| MAOU | + | 10,59 | 1546 | 16.7 | 04 | 04 | 0103 | alive | |
| RIGO | + | 8,04 | 262 | 17.4 | 04 | 13 | 0103 | alive | |
| SEND | + | 12,15 | 4549 | 15.6 | * | * | * | dead | |
| TROL | + | 10,31 | 1270 | 15.4 | * | * | * | dead | |
| LOG | + | 11,83 | 3649 | 16.7 | 04 | 13 | * | alive | |

Table 6 summarizes HLA-DR data obtained with microarray technique, real-time PCR (qPCR) or genotyping in relation with outcome.

### Validation by real time PCR

Among 48 samples, only 3 results were false in comparison with HLA-DRB4 real time PCR data (Table 6). For the remaining samples, real time PCR data fitted well with those obtained with microarray for the best 3 genes tested with both methods (r=0.083, p < 0.0003 for HLA-DRB4; r=0.60, p<0.002 for THBS1, r=0.68, p<0.0001 for AREG). As found from microarray data, the HLA-DRB4 gene expression measured by real time PCR strongly related with outcome (Spearman test; p< 0.0001).

### HLA-DR genotyping

It is known that the presence of the gene HLA-DRB4 is associated with specific alleles on the gene HLA-DRB1 (Bodmer et al., 1992; Nepom and Erlich, 1991). The alleles related to the presence of HLA-DRB4 are B1*04, B1*07 and B1*09, a relation confirmed in the population herein studied. Interestingly, HLA-DRB4 was always present when it was expressed with PCR, and was always absent when it was not expressed in PCR. As a consequence, HLA-DRB4 gene presence or not strongly related with patient outcome (p= 0.041). Very few discrepancies between microarray and PCR were observed. When present, it can result from the presence in genotyping of a non functional allelic composition, counted as non-expressed HLA-DRB4 with microarray. This rare configuration suggests that it may be preferable to perform both genotyping and functional genomic (or only functional genomic). Three HLA-DRB4 alleles were observed to be associated to HLA-DRB1 alleles as follows: **B4*0103** was associated to 5 B1*0401, 1 B1*0404, 1 B1*0402, 1 B1*07 and 1 B1*09 alleles, **B4*0101** and **B4*01030102N** were associated to 5 B1*07 alleles. It was remarkable to note that for one patient (GAM 5066), HLA-DRB4 gene expression was found both with microarrays and PCR, although the genotyping revealed the null allele.

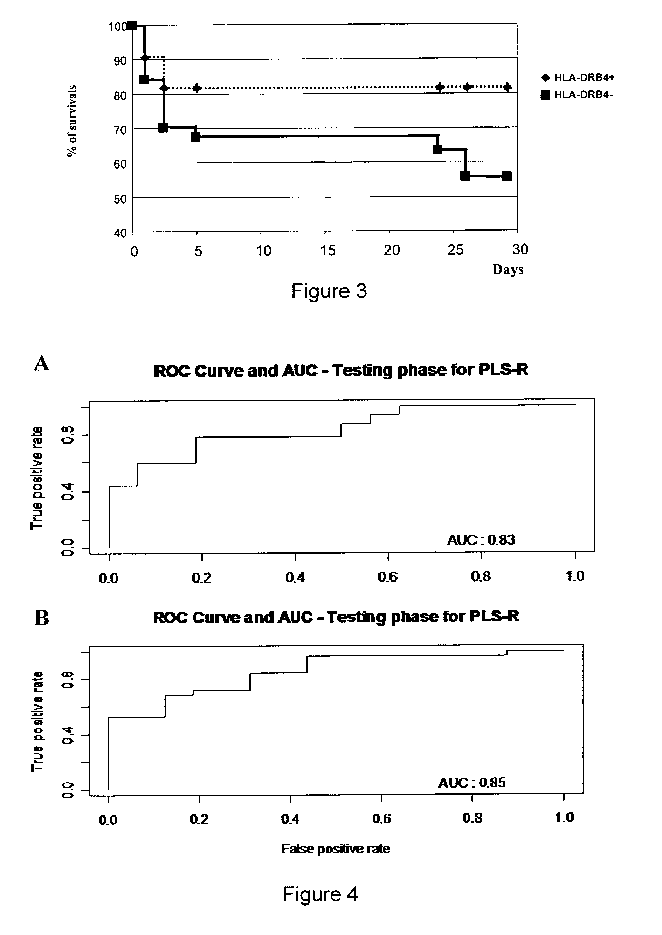

The expression and then the presence of HLA-DRB4 gene was associated with a good outcome in this septic shock population. Table 6 summarizes the results of HLA-DRB4 determination performed both with microarray and real time PCR in relation with outcome in the studied population. Figure 3 shows the outcome actuarial curve from day 0 to day 28 according to the B4 expression status. It shows a dramatic difference in mortality between HLA-DRB4+ and HLA-DRB4- gene expression at day 0.

### Predictive model:

Tables 7 and 8 give an estimation of the $\beta_j$ obtained by PLS regression.

Table 7 : estimation of the $\beta_j$ obtained by PLS regression For 29 selected sets of probes

| ID | SYMBOL | Estimated $\beta_j$ | mean | SD |
|---|---|---|---|---|
| 205239 at | AREG | -0,0346 | 4,0083 | 1,4529 |
| 202768 at | FOSB | 0,0303 | 6,0911 | 2,2296 |
| 201110 s_at | THBS1 | -0,0262 | 9,7647 | 1,5235 |
| 220146 at | TLR7 | 0,0249 | 4,0384 | 1,4833 |
| 238066 at | RBP7 | 0,0236 | 6,7761 | 1,6959 |
| 202087 s at | CTSL1 | -0,0245 | 8,2737 | 1,4496 |
| 210664 s at | TFPI | -0,0238 | 5,0884 | 1,4749 |
| 205992 s at | IL15 | 0,0213 | 5,7926 | 1,6720 |
| 214768 x_at | HLA-C | 0,0230 | 6,8021 | 2,1369 |
| 201109 s at | THBS1 | -0,0194 | 8,2284 | 1,7132 |
| 202203 s at | AMFR | 0,0174 | 5,1227 | 1,5190 |
| 224342 x_at | LOC96610 | 0,0214 | 6,0576 | 1,8032 |

(continued)

| ID | SYMBOL | Estimated $\beta_j$ | mean | SD |
|---|---|---|---|---|
| 239412 at | IRF5 | 0,0210 | 6,5024 | 1,8234 |
| 223565 at | MGC29506 | 0,0203 | 4,0536 | 1,8344 |
| 213258 at | TFPI | -0,0199 | 5,1292 | 1,6659 |
| 221286 s at | MGC29506 | 0,0191 | 6,7500 | 1,6989 |
| 244774 at | PHACTR2 | -0,0186 | 4,9173 | 1,7007 |
| 226733 at | PFKFB2 | -0,0186 | 8,8393 | 1,8872 |
| 205220 at | GPR109B | 0,0195 | 4,7479 | 2,4817 |
| 209728 at | HLA-DRB4 | 0,0192 | 5,7642 | 4,0364 |
| 208168 s at | CHIT1 | -0,0184 | 6,9050 | 2,3099 |
| 215775 at | THBS1 | -0,0181 | 4,7631 | 1,8197 |
| 217179 x_at | LOC96610 | 0,0178 | 6,4118 | 1,9703 |
| 228176 at | EDG3 | 0,0192 | 5,0845 | 2,0211 |
| 234764 x_at | IGLV1-44 | 0,0165 | 7,9806 | 1,8912 |
| 217502 at | IFIT2 | 0,0176 | 4,4420 | 1,9821 |
| 214453 s at | IFI44 | 0,0181 | 6,9163 | 1,9879 |
| 227609 at | EPSTI1 | 0,0173 | 6,3832 | 2,4141 |
| 206641 at | TNFRSF17 | 0,0162 | 4,8530 | 2,5299 |

Table 8 : estimation of the $\beta_j$ obtained by PLS regression For 8 selected sets of probes

| ID | SYMBOL | Estimated $\beta_j$ | mean | SD |
|---|---|---|---|---|
| 209728 at | HLA-DRB4 | 0,0581 | 5,7642 | 4,0364 |
| 202768 at | FOSB | 0,0916 | 6,0911 | 2,2296 |
| 205239 at | AREG | -0,1044 | 4,0083 | 1,4529 |
| 205220 at | GPR109B | 0,0589 | 4,7479 | 2,4817 |
| 238066 at | RBP7 | 0,0712 | 6,7761 | 1,6959 |
| 201110 s at | THBS1 | -0,0790 | 9,7647 | 1,5235 |
| 220146 at | TLR7 | 0,0751 | 4,0384 | 1,4833 |
| 202087 s at | CTSL1 | -0,0739 | 8,2737 | 1,4496 |

As an example for the model including the 8 selected probe sets, the score $\hat{y}$ for a new patient is obtained as follows:

$$Score = \hat{y} =$$

$$0.0581 \times \left( \frac{HLADRB4 - 5.7642}{4.0364} \right) + 0.0916 \times \left( \frac{FOSB - 6.0911}{2.2296} \right) -$$

$$0.1044 \times \left( \frac{AREG - 4.0083}{1.4529} \right) + 0.0589 \times \left( \frac{GPR109B - 4.7479}{2.4817} \right) +$$

$$0.0712 \times \left( \frac{RBP7 - 6.7761}{1.6959} \right) - 0.0790 \times \left( \frac{THBS1 - 9.7647}{1.5235} \right) +$$

$$0.0751 \times \left( \frac{TLR7 - 4.0384}{1.4833} \right) - 0.0739 \times \left( \frac{CTLS1 - 8.2737}{1.4496} \right)$$

From the gene expression profile of a new patient, this equation can be applied to predict his/her outcome. The equation will refine along time by increasing the size of the learning set of patients to compute the model.

The ROC (Receiving Operator Characteristics) curves generated by cross validation provide indications for sensitivity and specificity. These curves are shown in figure 4.

The thresholds are those providing the best precision or sensibility.

For the equation considering 29 sets of probes, two examples of contingency tables are presented below:

Table 9: contingency table for the best precision (83%), and a sensibility of 69% with threshold=-0.24 (specificity = 90%)

|  |  | PREDICTED | |
|---|---|---|---|
|  |  | Dead | Alive |
| OBSERVED | Dead | 11 | 5 |
|  | Alive | 3 | 29 |

Table 10: contingency table for the best couple precision sensibility (resp. 75 and 81%) with threshold=0.04 (specificity = 72%)

|  |  | PREDICTED | |
|---|---|---|---|
|  |  | Dead | Alive |
| OBSERVED | Dead | 13 | 3 |
|  | Alive | 9 | 23 |

For the model with 8 sets of probes, the best couple precision sensibility (resp. 75 and 88%) with threshold=0.03 gives the following table 11 (specificity = 72 %).

|  |  | PREDICTED | |
|---|---|---|---|
|  |  | Dead | Alive |
| OBSERVED | Dead | 14 | 2 |
|  | Alive | 10 | 22 |

In practice, a new patient will be classified in term of prognosis as follows: for a score $\hat{y} > 0.03$ (8 sets of probes footprint), the patient will have a high probability to survive and will not require costly specific treatment(s).

**Discussion**

Until now, no biomarkers have been proven relevant to well predict outcome in septic shock patients, who have a severe prognosis (40-50% mortality rate). Such marker(s) would be crucial for the initial triage to adapt the supportive care and resources and to decide if high tech and expensive drugs have to be given or investigated. Except for scoring systems such as SAPS II or SOFA score determination, no relations were found with outcome. The interest to use these scores is hampered by the necessity to wait 24 hours for quoting, a delay that might be long to make decisions.

The medical community is convinced that the therapeutic window between the septic shock onset and the non-reversibility of tissue lesions is short. It has been shown that aggressive resuscitation early performed in septic shock patients reduced mortality about 40% (Rivers et al., 2001), whereas the same strategy applied after 24 hrs did not change the prognosis. As a consequence, effectiveness of high tech drugs in septic shock such as Activated Protein C (APC) is time dependent. A rapid determination of patients with high risk of mortality is then warranted. Moreover, the future development of new drugs and the performance of clinical trials would be helped if only the patients with a poor prognosis are included.

Until now, the gene by gene or protein by protein approach had failed to provide a picture of septic shock with therapeutic consequences. This failure results from multifactorial causes such as the extreme complexity of the cellular modifications, the system interactions and redundancy, the impact of micro-organisms and the comorbidity. Because of that, it seems suitable to use a multiplexic approach as it is possible with micro-array technique.

The suitable tissue(s) for such approach could concern all the failing organs and blood cells. Tissue samples have limitations since it is ethically difficult to perform biopsies in patients in severe conditions especially for coagulation. In addition, organ failures occur after a certain delay that might be too long to make decisions. Blood cell study sounds then reasonable, since circulating immune cells are activated and are signalling the response of remote tissue cells. Although the number of cells is small compared to tissue samples, it can be drawn easily and the quantity of RNA material for micro-array becomes smaller than before. This approach has been used for several microarray studies in healthy volunteers (Calvano et al., 2005).

The septic shock population studied had a high risk of mortality (34%) as shown by the expected mortality related to the SAPS II.

The expression of gene HLA-DRB4 appeared to be strongly associated with a good outcome in septic shock. This molecule belongs to the MHC class II system implicated in the presentation of antigen by antigen presenting cells (monocyte, macrophage, B lymphocyte or dendritic cells) to T lymphocyte during the early immune response. HLA-DRB4 gene is located on a highly polymorphic locus (6p21) associated to numerous auto-immune disorders such as multiple sclerosis and rheumatoid arthritis (Holoshitz et al., 1992). It is well established that polymorphisms for this locus include presence or absence of the DRB4. Thus, 50% of the caucasian population do not have any copy of DRB4 gene in their genomes. Such a characteristic could explain the massive difference observed in expression level between groups. HLA-DRB genes are all located in the same cluster and are functionally redundant. Only 4 different genes encode functional proteins (DRB1, DRB3, DRB4 and DRB5). In addition, 3 pseudogenes are known. If DRB1 is found constitutive in human, the other genes are only present in some specific haplogroups. This important observation led the inventors to proceed to an extensive genotyping of this locus (Table 6). Gathering data from microarray, genotyping and qPCR, they were able to separate patients expressing a functional HLA-DRB4 protein from those who did not. This analysis shows that patients bearing/expressing DRB4 gene had a largely better chance to survive septic shock. As we can see in figure 4, 82% of septic patients expressing HLA-DRB4 are still alive 4 weeks after the inclusion compared to less than 54% in DRB4 negative patients. It is however difficult to make a mechanistic link between the HLA-DRB4 protein and survival in sepsis, since until now little attention has been brought to this molecule. One hypothesis would be related to the incapacity to present superantigen that may limit the inflammatory response (Herman et al., 1990).

Since the invention will have to be used to classify new patient, it appeared crucial to propose a method for this purpose. The proposed model based on the fluorescence of 29 (or 8) probe sets of interest measured by the microarray technique allowed bulding a classification model which will lead to determine a threshold value of -0.24 (or 0.03 for 8 probe sets) suitable to classify new patients. In medical diagnosis applications, precision is not the main issue; sensitivity and specificity are far more relevant because of different misclassification costs. Diagnostic and therapeutic decision correspond to a modulation of a sensitivity level, this decision is very important for the outcome prediction because of the unbalanced classification cost: sensibility is far more important than specificity since disregarding a high death risk patient is a severe error. The proposed models show a high sensitivity in detriment to the specificity, which means a good estimation of death risk: we can afford a low specificity because the potential false diagnosis will not have detrimental consequences, as the proposed treatment does not have any severe adverse consequences. The proposed models appear to fit well with these goals.

Finally, the models will be refined along time since the number of additional patients will help to better define coefficient of the models.

In conclusion, application of a pangenomic microarray technique to white blood cells after elimination of mature PMNs in septic shock patients provided a prognostic footprint. At day 0 of septic shock, 24 genes (detected by 29 sets of probes) were demonstrated to predict outcome within the next 4 weeks. Among these, HLA-DRB4 is a strong determinant, with an expression which is associated with a good prognosis. The large difference in expression between survivals and non survivals has genotyping correspondence: expression is always related to the constitutive presence of the gene, whereas the under-expression or absence of expression always corresponded to the constitutive absence of this gene. The expression level of the 28 other selected genes is important to determine, especially for negative expression of HLA-DRB4.

**REFERENCES**

Annane, D., and Bellissant, E. (2000). Prognostic value of cortisol response in septic shock. Jama 284, 308-309.

Belikova, I,, Lukaszewicz, A. C., Faivre, V., Damoisel, C., Singer, M., and Payen, D. (2007). Oxygen consumption of human peripheral blood mononuclear cells in severe human sepsis. Crit Care Med 35, 2702-2708.

Bernard, G. R., Vincent, J. L., Laterre, P. F., LaRosa, S. P., Dhainaut, J. F., Lopez-Rodriguez, A., Steingrub, J. S., Garber, G. E., Helterbrand, J. D., Ely, E. W., and Fisher, C. J., Jr. (2001). Efficacy and safety of recombinant human activated protein C for severe sepsis. N Engl J Med 344, 699-709.

Bodmer, J. G., Marsh, S. G., Albert, E. D., Bodmer, W. F., Dupont, B., Erlich, H. A., Mach, B., Mayr, W. R., Parham, P., Sasazuki, T., and et al. (1992). Nomenclature for factors of the HLA system, 1991. Eur J Immunogenet 19, 327-344.

Calvano, S. E., Xiao, W., Richards, D. R., Felciano, R. M., Baker, H. V., Cho, R. J., Chen, R. O., Brownstein, B. H., Cobb, J. P., Tschoeke, S. K., et al. (2005). A network-based analysis of systemic inflammation in humans. Nature 437, 1032-1037.

Dellinger, R. P., Levy, M. M., Carlet, J. M., Bion, J., Parker, M. M., Jaeschke, R., Reinhart, K., Angus, D. C., Brun-Buisson, C., Beale, R., et al. (2008). Surviving Sepsis Campaign: international guidelines for management of severe sepsis and septic shock: 2008. Crit Care Med 36, 296-327.

Docke, W. D., Randow, F., Syrbe, U., Krausch, D., Asadullah, K., Reinke, P., Volk, H. D., and Kox, W. (1997). Monocyte deactivation in septic patients: restoration by IFN-gamma treatment. Nat Med 3, 678-681.

Herman, A., Croteau, G., Sekaly, R. P., Kappler, J., and Marrack, P. (1990). HLA-DR alleles differ in their ability to present staphylococcal enterotoxins to T cells. J Exp Med 172, 709-717.

Holoshitz, J., Vila, L. M., Keroack, B. J., McKinley, D. R., and Bayne, N. K. (1992). Dual antigenic recognition by cloned human gamma delta T cells. J Clin Invest 89, 308-314.

Irizarry, R. A., Bolstad, B. M., Collin, F., Cope, L. M., Hobbs, B., and Speed, T. P. (2003). Summaries of Affymetrix GeneChip probe level data. Nucleic Acids Res 31, e15.

Nepom, G. T., and Erlich, H. (1991). MHC class-II molecules and autoimmunity. Annu Rev Immunol 9, 493-525.

Pachot, A., Barbalat, V., Marotte, H., Diasparra, J., Gouraud, A., Mougin, B., and Miossec, P. (2007). A rapid semi automated method for DNA extraction from dried-blood spots: application to the HLA-DR shared epitope analysis in rheumatoid arthritis. J Immunol Methods 328, 220-225.

Rivers, E., Nguyen, B., Havstad, S., Ressler, J., Muzzin, A., Knoblich, B., Peterson, E., and Tomlanovich, M. (2001). Early goal-directed therapy in the treatment of severe sepsis and septic shock. N Engl J Med 345, 1368-1377.

Rosipal, R., Trejo, L.J. and Matthews, B. (2003) Kernel PLS-SVC for Linear and Nonlinear Classification. In Proceedings of the Twentieth International Conference on Machine Learning (ICML'03), Washington DC, 640-647.

Tenenhaus, A., Giron, A., Viennet, E., Béra, M., Saporta, G. and Fertil, B. (2007). Kernel logistic PLS: A tool for supervised nonlinear dimensionality reduction and binary classification, 2007, 51 (9), 4083-4100.

Tenenhaus M., La régression PLS, Technip, 1998.

van 't Veer, L. J., Dai, H., van de Vijver, M. J., He, Y. D., Hart, A. A., Mao, M., Peterse, H. L., van der Kooy, K., Marton, M. J., Witteveen, A. T., et al. (2002). Gene expression profiling predicts clinical outcome of breast cancer. Nature 415, 530-536.

Vapnik, V. (1998). Statistical learning theory. Wiley, NY.

Venet, F., Tissot, S., Debard, A. L., Faudot, C., Crampe, C., Pachot, A., Ayala, A., and Monneret, G. (2007). Decreased monocyte human leukocyte antigen-DR expression after severe bum injury: Correlation with severity and secondary septic shock. Crit Care Med 35, 1910-1917.

Vincent, J. L., de Mendonca, A., Cantraine, F., Moreno, R., Takala, J., Suter, P. M., Sprung, C. L., Colardyn, F., and Blecher, S. (1998). Use of the SOFA score to assess the incidence of organ dysfunction/failure in intensive care units: results of a multicenter, prospective study. Working group on "sepsis-related problems" of the European Society of Intensive Care Medicine. Crit Care Med 26, 1793-1800 Boser, B., Guyon, I. et Vapnik, V. (1992). A training algorithm for optimal margin classifier. In Fifth Annual Workshop on Computational Learning Theory, 144-152. ACM.

Wold, S.; Martens, L. & Wold, H. The multivariate calibration problem in chemistry solved by the PLS method Proceedings Conf. Matrix Pencils, Ruhe A. & Kastrø m B, Lecture Notes in Mathematics, Springer Verlag, 1983, 286-293.

## Claims

1.  Use of at least one gene selected in the group consisting of HLA-DRB4, FOSB, GPR109B, RBP7, TLR7, IL15, HLA-C, AMFR, LOC96610, IRF5, MGC29506, EDG3, IGLV1-44, IFIT2, IFI44, EPSTI1, TNFRSF17, AREG, THBS1, CTLS1, TFPI, PHACTR2, PFKFB2 and CHIT1, as a prognosis marker for a patient in severe sepsis with at least two organ failures.

2.  The use of claim 1, wherein said at least one gene is selected in the group consisting of HLA-DRB4, AREG, FOSB, GPR109B, RBP7, TLR7, THBS1 and CTLS1.

3.  The use of claim 1 or claim 2, wherein said at least one gene is selected in the group consisting of HLA-DRB4, AREG and FOSB.

4. A method for *in vitro* establishing a prognosis for a subject in severe sepsis with at least two organ failures, comprising the following steps:

   (i) from a sample from said subject, obtaining an expression profile of a set of genes comprising at least two genes selected in the group consisting of HLA-DRB4, FOSB, GPR109B, RBP7, TLR7, IL15, HLA-C, AMFR, LOC96610, IRF5, MGC29506, EDG3, IGLV1-44, IFIT2, IFI44, EPSTI1, TNFRSF17, AREG, THBS1, CTLS1, TFPI, PHACTR2, PFKFB2 and CHIT1; and
   (ii) comparing said obtained expression profile to one or two reference expression profiles to establish a prognosis for said subject.

5. The method according to claim 4, comprising the following steps:

   (i) the expression levels *of n* ($n \geq 2$) genes are measured in a biological sample from said patient, wherein at least two of these genes are selected in the group consisting of HLA-DRB4, FOSB, GPR109B, RBP7, TLR7, IL15, HLA-C, AMFR, LOC96610, IRF5, MGC29506, EDG3, IGLV1-44, IFIT2, IFI44, EPSTI1, TNFRSF17, AREG, THUS1, CTLS1, TFPI, PHACTR2, PFKFB2 and CHIT1;
   (ii) a score is calculated as follows:

$$Score = \hat{y} = \sum_{j=1}^{n} \hat{\beta}_j \widetilde{X}_j \qquad (1)$$

   wherein $\widetilde{X}_j$ ($j$ = 1 to $n$) are the expression levels of said genes measured in said biological sample, and $\beta_j$ ($j$ = 1 to $n$) are calculated regression coefficients;
   (iii) the score obtained in step (ii) is interpreted by comparing it to a predetermined threshold.

6. The method according to claim 4 or claim 5, wherein at least one of the genes selected in step (i) is selected in the group consisting of HLA-DRB4, AREG and FOSB.

7. The method according to any of claims 4 to 6, wherein the set of genes selected in step (i) comprises HLA-DRB4, AREG and FOSB.

8. The method according to any of claims 4 to 6, wherein the set of genes selected in step (i) comprises HLA-DRB4, AREG, FOSB, GPR109B, RBP7, TLR7, THBS1 and CTLS1.

9. The method according to any of claims 4 to 8, wherein the set of genes selected in step (i) comprises HLA-DRB4, FOSB, GPR109B, RBP7, TLR7, IL15, HLA-C, AMFR, LOC96610, IRF5, MGC29506, EDG3, IGLV1-44, IFIT2, IFI44, EPSTI1, TNFRSF17, AREG, THBS1, CTLS1, TFPI, PHACTR2, PFKFB2 and CHIT1.

10. The use of any of claims 1 to 3, or the method according to any of claims 4 to 9, for establishing a prognosis for a patient in septic shock with at least one additional organ failure.

11. The method according to any of claims 4 to 10, wherein said biological sample is (i) a whole blood sample or (ii) a blood fraction comprising or consisting ofperipheral blood mononuclear cells, or (iii) a blood fraction comprising or consisting of white blood cells after removal of mature granulocytes.

12. A method for determining if a subject in severe sepsis with at least two organ failures can benefit from the administration of a medicinal product, comprising a step of *in vitro* determining if said patient expresses HLA-DRB4.

13. The method according to claim 12, wherein the absence of HLA-DRB4 expression indicates that administration of Activated Protein C to said patient is appropriate.

14. The method of claim 12 or claim 13, wherein in the absence of HLA-DRB4 expression, up-regulation of one or several genes selected amongst FOSB, GPR109B, RBP7, TLR7, IL15, HLA-C, AMFR, LOC96610, IRF5, MGC29506, EDG3, IGLV1-44, IFIT2, IFI44, EPSTI1 and TNFRSF17, and/or down-regulation of one or several genes selected amongst AREG, THBS1, CTLS1, TFPI, PHACTR2, PFKFB2 and CHIT1 indicate(s) that said patient will be a good responder to Activated Protein C.

15. A method for *in vitro* evaluating the efficiency of a pharmaceutical treatment administered to a subject in severe sepsis with at least two organ failures, comprising the following steps:

(i) from a sample from said subject obtained before the beginning of said pharmaceutical treatment, determining an expression profile of a set of genes comprising at least two genes selected in the group consisting of HLA-DRB4, FOSB, GPR109B, RBP7, TLR7, IL15, HLA-C, AMFR, LOC96610, IRF5, MGC29506, EDG3, IGLV1-44, IFIT2, IFI44, EPSTI1, TNFRSF17, AREG, THBS1, CTLS1, TFPI, PHACTR2, PFKFB2 and CHIT1;
(ii) from at least another sample from said subject, obtained after the beginning of said pharmaceutical treatment, determining an expression profile of the same set of genes as in (i) ;
(iii) comparing said obtained expression profiles.

16. The method according to claim 15, wherein up-regulation of one or several genes selected amongst FOSB, GPR109B, RBP7, TLR7, IL15, HLA-C, AMFR, LOC96610, IRF5, MGC29506, EDG3, IGLV1-44, IFIT2, IFI44, EPSTI1 and TNFRSF17, and/or down-regulation of one or several genes selected amongst AREG, THBS1, CTLS1, TFPI, PHACTR2, PFKFB2 and CHIT1 following the beginning of the pharmaceutical treatment indicate(s) that said treatment has been beneficial to the patient.

17. A method for selecting subjects to be enrolled in a clinical trial for evaluating a medicinal product in the treatment of severe sepsis with at least two organ failures, comprising a step of *in vitro* establishing a prognosis for said subjects, by a method according to any of claims 4 to 11.

18. The method of claim 17, wherein the subjects enrolled in the clinical trial are those who have a poor prognosis.

19. A kit for performing the method according to any of claims 4 to 18, comprising one or several pairs of primers, wherein each pair of primers is specific for a gene selected amongst HLA-DRB4, AREG and FOSB.

20. The kit of claim 20, further comprising one or several additional pairs of primers, wherein each pair of primers is specific for one sequence selected in the group consisting of 18S rRNA, GPR109B, RBP7, TLR7, IL15, HLA-C, AMFR, LOC96610, IRF5, MGC29506, EDG3, IGLV1-44, IFIT2, IFI44, EPSTI1, TNFRSF17, THBS 1, CTLS 1, TFPI, PHACTR2, PFKFB2 and CHIT1 genes.

21. The kit of claim 19 or claim 20, which further comprises reagents and/or enzymes for performing amplification reactions.

22. A kit for performing the method according to any of claims 4 to 18, comprising a chip enabling hybridization of nucleic acids specific for at least two genes selected in the group consisting of HLA-DRB4, AREG, FOSB, GPR109B, RBP7, TLR7, IL15, HLA-C, AMFR, LOC96610, IRF5, MGC29506, EDG3, IGLV1-44, IFIT2, IFI44, EPSTI1, TNFRSF17, THBS1, CTLS1, TFPI, PHACTR2, PFKFB2 and CHIT1.

23. The kit of any of claims 19 to 22, which further comprises reagents for PBMC isolation.

Figure 1

Figure 2

Figure 3

A

**ROC Curve and AUC – Testing phase for PLS-R**

B

**ROC Curve and AUC – Testing phase for PLS-R**

Figure 4

Europäisches
Patentamt

European
Patent Office

Office européen
des brevets

**EUROPEAN SEARCH REPORT**

Application Number

EP 08 29 0090

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2006/079760 A (BIOMERIEUX SA [FR]; PACHOT ALEXANDRE [FR]; MONNERET GUILLAUME [FR]; LE) 3 August 2006 (2006-08-03) p. 1-5, example 1, p. 26, l. 14 ----- | 1-15,17, 18 | INV. C12Q1/68 |
| X | LIVADITI ET AL: "Neutrophil CD64 expression and serum IL-8: Sensitive early markers of severity and outcome in sepsis" CYTOKINE, ACADEMIC PRESS LTD, PHILADELPHIA, PA, US, vol. 36, no. 5-6, 1 December 2006 (2006-12-01), pages 283-290, XP022020204 ISSN: 1043-4666 abstract, p. 284, col. 2 "Material and methods", Tab 3. ----- | 1-15,17, 18 | |
| A | CZERWONY GRIT ET AL: "Differential surface expression of HLA-DRB1 and HLA-DRB4 among peripheral blood cells of DR4 positive individuals" HUMAN IMMUNOLOGY, vol. 60, no. 1, January 1999 (1999-01), pages 1-9, XP002488317 ISSN: 0198-8859 abstract and p. 1, col. 1 ----- | 1-15, 17-23 | |
| A | CAILLE VINCENT ET AL: "Histocompatibility leukocyte antigen-D related expression is specifically altered and predicts mortality in septic shock but not in other causes of shock." SHOCK (AUGUSTA, GA.) DEC 2004, vol. 22, no. 6, December 2004 (2004-12), pages 521-526, XP002488318 ISSN: 1073-2322 abstract, p. 521, col. 1 "Material and methods", and fig. 1 ----- | 1-15, 17-23 | |

**TECHNICAL FIELDS SEARCHED (IPC)**

C12Q

-/--

~~The present search report has been drawn up for all claims~~

| Place of search | Date of completion of the search | Examiner |
|---|---|---|
| Munich | 22 July 2008 | Berillon-Lapopin, L |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons
.............................................................
& : member of the same patent family, corresponding
document

EPO FORM 1503 03.82 (P04C01)

Europäisches
Patentamt

European
Patent Office

Office européen
des brevets

## EUROPEAN SEARCH REPORT

**Application Number**

EP 08 29 0090

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | HELDT CHRISTIAN ET AL: "Differential expression of HLA class II genes associated with disease susceptibility and progression in rheumatoid arthritis." ARTHRITIS AND RHEUMATISM OCT 2003, vol. 48, no. 10, October 2003 (2003-10), pages 2779-2787, XP002488319 ISSN: 0004-3591 p. 2782 col. 1 and tab. 1 ----- | 19-21 | |
| X | SKUBITZ ET AL: "Differential gene expression identifies subgroups of ovarian carcinoma" TRANSLATIONAL RESEARCH, ELSEVIER, AMSTERDAM, NL, vol. 148, no. 5, 26 October 2006 (2006-10-26), pages 223-248, XP005742317 ISSN: 1931-5244 tab. III, p. 233. ----- | 22,23 | |
| A,D | BONE R C ET AL: "DEFINITIONS FOR SEPSIS AND ORGAN FAILURE AND GUIDELINES FOR THE USE OF INNOVATIVE THERAPIES IN SEPSIS" CHEST, THE COLLEGE, CHICAGO, IL, US, vol. 101, no. 6, 1 June 1992 (1992-06-01), pages 1644-1655, XP009044010 ISSN: 0012-3692 * table 1 * ----- | 1-15, 17-23 | TECHNICAL FIELDS SEARCHED (IPC) |
| A,D | VINCENT J-L ET AL: "THE SOFA (SEPSIS-RELATED ORGAN FAILURE ASSESSMENT) SCORE TO DESCRIBE ORGAN DYSFUNCTION/FAILURE" INTENSIVE CARE MEDICINE, BERLIN, DE, vol. 22, no. 7, 1 July 1996 (1996-07-01), pages 707-710, XP009037295 ISSN: 0342-4642 * table 3 * ----- | 1-15, 17-23 | |

~~The present search report has been drawn up for all claims~~

| Place of search | Date of completion of the search | Examiner |
|---|---|---|
| Munich | 22 July 2008 | Berillon-Lapopin, L |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

  ...........................................................................................................
& : member of the same patent family, corresponding
document

EPO FORM 1503 03.82 (P04C01)

Europäisches
Patentamt

European
Patent Office

Office européen
des brevets

Application Number

EP 08 29 0090

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

see sheet B

☐ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☒ None of the further search fees have been paid within the fixed time limit. The present European search report has been drawn up for those parts of the European patent application which relate to the invention first mentioned in the claims, namely claims:

1-11, 15, 17-23 (partly); 12-14 (completely)

☐ The present supplementary European search report has been drawn up for those parts of the European patent application which relate to the invention first mentioned in the claims (Rule 164 (1) EPC).

Europäisches
Patentamt

European
Patent Office

Office européen
des brevets

**LACK OF UNITY OF INVENTION**
**SHEET B**

The Search Division considers that the present European patentapplication does not comply with the requirements of unity of invention and relates to severalinventions or groups of inventions, namely:

Invention 1: claims 1-11, 15, 17-23 (partly) and 12-14 (completely)

        concern the use of HLA-DBR4 as prognosis markers for a patient in septic shock (i.e. in severe sepsis with at least 2 organ failures, see preliminary remark below). Said claims concern also methods: for prognosis septic shock, for determining if a subject can benefit for a medical product, for evaluating the efficicency of a treatment, and for selecting a subject for a clinical trial for evaluating a medicinal product, all by monitoring the expression level of HLA-DBR4. Said claims concern also kits comprising primers and a chip having nucleic acids specific for the said maker.
        ---

Invention 2: claims 1-11,and 15-23 (partly)

        concern the use of FOSB as prognosis markers for a patient in septic shock. Said claims concern also methods for prognosis septic shock, for evaluating the efficicency of a treatment, and for selecting a subject for a clinical trial for evaluating a medicinal product, all by monitoring the expression level of FOSB. Said claims concern also kits comprising primers and chip having nucleic acids specific for the said maker.
        ---

Inventions 3-5: claims 1, 2, 4, 5, 8-11, 15-18, 22 and 23 (partly)

        concern the use of GPR109B, RBP7 and TLR7 as prognosis markers for a patient in septic shock. Said claims concern also methods for prognosis septic shock, for evaluating the efficicency of a treatment, and for selecting a subject for a clinical trial for evaluating a medicinal product, all by monitoring the expression level of GPR109B, RBP7 and TLR7. Said claims concern also a kit comprising a chip having nucleic acids specific for the said makers.
        ---

Inventions 6-17: claims 1, 4, 5, 9-11, 15-18, 22 and 23 (partly)

        concern the use of IL15, HLA-C, AMFR, LOC96610, IRF5, MGC29506, EDG3, IGLVI-44, IFIT2, IFI44, EPST11 and TNFRSF17 as prognosis markers for a patient in septic shock. Said claims concern also methods for prognosis septic shock, for evaluating the efficicency of a treatment, and for selecting a subject for a clinical trial for evaluating a medicinal product, all by monitoring the expression level of IL15, HLA-C, AMFR, LOC96610, IRF5, MGC29506, EDG3, IGLVI-44, IFIT2, IFI44, EPST11 and TNFRSF17. Said claims concern also a kit comprising a chip having nucleic acids specific for the said makers.

Europäisches
Patentamt

European
Patent Office

Office européen
des brevets

**LACK OF UNITY OF INVENTION
SHEET B**

Application Number

EP 08 29 0090

The Search Division considers that the present European patentapplication does not comply with the
requirements of unity of invention and relates to severalinventions or groups of inventions, namely:

---

Invention 18: claims 1-11,and 15-23 (partly)

  concern the use of  as prognosis AREG marker for a patient
  in septic shock. Said claims concern also methods for
  prognosis septic shock, for evaluating the efficicency of a
  treatment, and for selecting a subject for a clinical trial
  for evaluating a medicinal product, all by monitoring the
  expression level of AREG. Said claims concern also kits
  comprising primers and chip having nucleic acids specific
  for the said maker.

  ---

 Inventions 19 and 20: claims 1, 2, 4, 5, 8-11, 15-18, 22 and 23
  (partly)

  concern the use of THBS1 and CTLS1 as prognosis markers for
  a patient in septic shock. Said claims concern also methods
  for prognosis septic shock, for evaluating the efficicency
  of a treatment, and for selecting a subject for a clinical
  trial for evaluating a medicinal product, all by monitoring
  the expression level of THBS1 and CTLS1. Said claims concern
  also a kit comprising a chip having nucleic acids specific
  for the said makers.

  ---

Inventions 21-24: claims 1, 4, 5, 9-11, 15-18, 22 and 23 (partly)

  concern the use of TFPI, PHACTR2, PFKFB2 and CHIT1 as
  prognosis markers for a patient in septic shock. Said claims
  concern also methods for prognosis septic shock, for
  evaluating the efficicency of a treatment, and for selecting
  a subject for a clinical trial for evaluating a medicinal
  product, all by monitoring the expression level of TFPI,
  PHACTR2, PFKFB2 and CHIT1. Said claims concern also a kit
  comprising a chip having nucleic acids specific for the said
  makers.

  ---

# EP 2 085 486 A1

## ANNEX TO THE EUROPEAN SEARCH REPORT
## ON EUROPEAN PATENT APPLICATION NO.

EP 08 29 0090

22-07-2008

| Patent document cited in search report | Publication date | Patent family member(s) | | Publication date |
|---|---|---|---|---|
| WO 2006079760 A | 03-08-2006 | EP | 1844159 A1 | 17-10-2007 |
| | | FR | 2881437 A1 | 04-08-2006 |
| | | JP | 2008528018 T | 31-07-2008 |

EPO FORM P0459

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **Annane, D. ; Bellissant, E.** Prognostic value of cortisol response in septic shock. *Jama,* 2000, vol. 284, 308-309

- **Belikova, I ; Lukaszewicz, A. C. ; Faivre, V. ; Damoisel, C. ; Singer, M. ; Payen, D.** Oxygen consumption of human peripheral blood mononuclear cells in severe human sepsis. *Crit Care Med,* 2007, vol. 35, 2702-2708

- **Bernard, G. R. ; Vincent, J. L. ; Laterre, P. F. ; LaRosa, S. P. ; Dhainaut, J. F. ; Lopez-Rodriguez, A. ; Steingrub, J. S. ; Garber, G. E. ; Helterbrand, J. D. ; Ely, E. W.** Efficacy and safety of recombinant human activated protein C for severe sepsis. *N Engl J Med,* 2001, vol. 344, 699-709

- **Bodmer, J. G. ; Marsh, S. G. ; Albert, E. D. ; Bodmer, W. F. ; Dupont, B. ; Erlich, H. A. ; Mach, B. ; Mayr, W. R. ; Parham, P. ; Sasazuki, T. et al.** Nomenclature for factors of the HLA system. *Eur J Immunogenet,* 1992, vol. 19, 327-344

- **Calvano, S. E. ; Xiao, W. ; Richards, D. R. ; Felciano, R. M. ; Baker, H. V. ; Cho, R. J. ; Chen, R. O. ; Brownstein, B. H. ; Cobb, J. P. ; Tschoeke, S. K. et al.** A network-based analysis of systemic inflammation in humans. *Nature,* 2005, vol. 437, 1032-1037

- **Dellinger, R. P. ; Levy, M. M. ; Carlet, J. M. ; Bion, J. ; Parker, M. M. ; Jaeschke, R. ; Reinhart, K. ; Angus, D. C. ; Brun-Buisson, C. ; Beale, R. et al.** Surviving Sepsis Campaign: international guidelines for management of severe sepsis and septic shock: 2008. *Crit Care Med,* 2008, vol. 36, 296-327

- **Docke, W. D. ; Randow, F. ; Syrbe, U. ; Krausch, D. ; Asadullah, K. ; Reinke, P. ; Volk, H. D. ; Kox, W.** Monocyte deactivation in septic patients: restoration by IFN-gamma treatment. *Nat Med,* 1997, vol. 3, 678-681

- **Herman, A. ; Croteau, G. ; Sekaly, R. P. ; Kappler, J. ; Marrack, P.** HLA-DR alleles differ in their ability to present staphylococcal enterotoxins to T cells. *J Exp Med,* 1990, vol. 172, 709-717

- **Holoshitz, J. ; Vila, L. M. ; Keroack, B. J. ; McKinley, D. R. ; Bayne, N. K.** Dual antigenic recognition by cloned human gamma delta T cells. *J Clin Invest,* vol. 89, 308-314

- **Irizarry, R. A. ; Bolstad, B. M. ; Collin, F. ; Cope, L. M. ; Hobbs, B. ; Speed, T. P.** Summaries of Affymetrix GeneChip probe level data. *Nucleic Acids Res,* 2003, vol. 31, e15

- **Nepom, G. T. ; Erlich, H.** MHC class-II molecules and autoimmunity. *Annu Rev Immunol,* 1991, vol. 9, 493-525

- **Pachot, A. ; Barbalat, V. ; Marotte, H. ; Diasparra, J. ; Gouraud, A. ; Mougin, B. ; Miossec, P.** A rapid semi automated method for DNA extraction from dried-blood spots: application to the HLA-DR shared epitope analysis in rheumatoid arthritis. *J Immunol Methods,* 2007, vol. 328, 220-225

- **Rivers, E. ; Nguyen, B. ; Havstad, S. ; Ressler, J. ; Muzzin, A. ; Knoblich, B. ; Peterson, E. ; Tomlanovich, M.** Early goal-directed therapy in the treatment of severe sepsis and septic shock. *N Engl J Med,* 2001, vol. 345, 1368-1377

- **Rosipal, R. ; Trejo, L.J. ; Matthews, B.** Kernel PLS-SVC for Linear and Nonlinear Classification. *Proceedings of the Twentieth International Conference on Machine Learning,* 2003, 640-647

- **Tenenhaus, A. ; Giron, A. ; Viennet, E. ; Béra, M. ; Saporta, G. ; Fertil, B.** *Kernel logistic PLS: A tool for supervised nonlinear dimensionality reduction and binary classification,* 2007, vol. 51 (9), 4083-4100

- **Tenenhaus M.** *La régression PLS, Technip,* 1998

- **van 't Veer, L. J. ; Dai, H. ; van de Vijver, M. J. ; He, Y. D. ; Hart, A. A. ; Mao, M. ; Peterse, H. L. ; van der Kooy, K. ; Marton, M. J. ; Witteveen, A. T. et al.** Gene expression profiling predicts clinical outcome of breast cancer. *Nature,* 2002, vol. 415, 530-536

- **Vapnik, V.** Statistical learning theory. Wiley, 1998

- **Venet, F. ; Tissot, S. ; Debard, A. L. ; Faudot, C. ; Crampe, C. ; Pachot, A. ; Ayala, A. ; Monneret, G.** Decreased monocyte human leukocyte antigen-DR expression after severe bum injury: Correlation with severity and secondary septic shock. *Crit Care Med,* 2007, vol. 35, 1910-1917

- **Vincent, J. L. ; de Mendonca, A. ; Cantraine, F. ; Moreno, R. ; Takala, J. ; Suter, P. M. ; Sprung, C. L. ; Colardyn, F. ; Blecher, S.** Use of the SOFA score to assess the incidence of organ dysfunction/failure in intensive care units: results of a multicenter, prospective study. Working group on ''sepsis-related problems'' of the European Society of Intensive Care Medicine. *Crit Care Med,* 1998, vol. 26, 1793-1800

- A training algorithm for optimal margin classifier. **Boser, B. ; Guyon, I. ; Vapnik, V.** Fifth Annual Workshop on Computational Learning Theory. ACM, 1992, 144-152

- The multivariate calibration problem in chemistry solved by the PLS method Proceedings Conf. Matrix Pencils. **Wold, S. ; Martens, L. ; Wold, H.** Lecture Notes in Mathematics. Springer Verlag, 1983, 286-293